# EUROPEAN PATENT APPLICATION

(11) **EP 3 093 654 A1**
(43) Date of publication of application: **16.11.2016**
(21) Application number: 16176170.5
(22) Date of filing: 06.06.2013
(51) Int. Cl.: G01N 27/327, G01N 33/49, A61B 8/06, G01P 5/18, G01F 1/64, G01N 11/04

(54) **ELECTROCHEMICAL TEST STRIP AND DETECTING METHOD FOR MEASURING THE FLOW TIME OF A SPECIMEN**

(30) Priority: 08.06.2012 TW 101120628; 08.06.2012 TW 101120587; 08.02.2013 TW 102105443
(62) Divisional of application: 13170749.9
(71) Applicant: HMD BioMedical Inc., 30548 Hsinchu County (TW)
(72) Inventor: HSU, Tien-Tsai, 30548 Hsinchu County (TW)
(74) Representative: Casalonga

(57) **Abstract**

A test strip and a detecting method are disclosed. The test strip can be used with an electrochemical instrument to accurately detect the flow time of a specimen. The test strip includes a specimen path, a first electrode, a redox reagent, a second electrode, and a reaction reagent. The redox reagent includes at least a redox pair. When the specimen enters the specimen path, the redox pair dissolves and generates an electrochemical redox reaction for obtaining a flow time of the specimen.

## Description

### BACKGROUND OF THE INVENTION

### 1. Field of the Invention

The present inventin relates to a test strip and a detecting method, and more particularly, to a test strip and a detecting methode which use a redox reagent to obtain a flow time of a specimen.

### 2. Description of the Related Art

Electrochemical bio-sensors have been widely adopted to find out the concentration of the analyte in a liquid specimen, such as blood or urine. There are many kinds of electrochemical bio-sensors, such as blood glucose sensors, cholesterol sensors, uric acid biosensors, and lactic acid biosensors. In particular, blood glucose sensors have become indispensable for diabetics. Generally, a blood glucose sensor is formed in a strip shape and comprises at least two electrodes such as a working electrode and a reference electrode for receving electrical signals proportional to the concentration of the blood glucose in a blood sample and transmitting the electrical signals to a blood glucose meter to indicate the blood glucose level.

On the other hand, full blood viscosity test can provide reliable reference to the diagnosis and treatment of pre- or post-thrombus in many research and clinical experiments. Numerous diseases such as hypertension, cardiopathy, coronary artery heart disease (CAHD), myocardial infarction, diabetic, malignant tumor and chronic hepatitis are highly related to blood viscosity. Blood viscosity coud be affected by the size, shape and hematocrit of red blood cells, which are the major part of the blood; although white blood cells and hematoblasts also could affect the blood viscosity; therefore, hematocrit (HCT) is the key factor in deciding the blood viscosity. Furthermore, when the blood viscosity increases, there will be more resistance in the blood, making it difficult to supply blood to the heart, brain, liver and kidney. As less blood is supplied, the symptoms could become worse; therefore, the blood viscosity has become an important index in monitoring the disease.

In order to measure the blood viscosity, there are many types of viscometer, such as capillary viscometer, cone and plate viscometer, coaxial cylinder viscometer, and pressure sensing viscometer, in which capillary viscometer is the most popular type. In a capillary viscometer, when parameters such volume, pressure difference, capillary diamter, and capillary length are constant, then the viscosity of the fluid is proportional to the time required to flow through the capillary; therefore, when the fluid is filled in the capillary, the viscosity of the fluid is obtained by using Poiseuiller's principle. However, there are some restrictions in using the capillary viscometer, for example, the capillary have to be straight, long and round in its cross section, the length to diamter ratio of the capillary usually needs to be more than 200, the diameter of the capillary is larger or equal to 1mm, and so on. Besides, the capillary viscometer has large equipment size, it needs a lot of sample volume to process and tends to require long reaction time; therefore, it is not easy to clean the capillary viscometeris, and it is not convenient to carry the capillary viscometer with the patient to detect the blood viscosity in real time. When it is necessary to obtain blood viscosity data from a group of people, it takes a great amount of time in detecting blood viscosity from each one of them and it requires to get enough specimens from them; therefore, it is inefficent and also not cost-effective.

Apart from the method for measuring blood viscosity as depicted above, US patent application US2007/0251836A1 disclosed an electrochemical sensor and method for analyzing a liquid sample, in which the electrochemical sensor comprises a channel for delivering the liquid sample; and a first conducting portion and a second conducting portion separated and exposed in the channel; wherein the first conducting portion generates a first pulse signal when it is contacted by the liquid sample, and the second conducting portion generates a second pulse signal when it is contacted by the liquid sample. The electrochemical sensor obtains viscosity of the liquid sample according to a time difference between the first and second pulse signals. Generally an electrochemical sensor provides a voltage no higher than 0.5V to save power and to avoid triggering unnecessary reactions; however, the signal could be very weak and unstable between the liquid sample such as blood and the electrodes, it could be covered by background noises and is difficult to be detected. Futhermore, the electrochemical sensor can be used to correct the concentration of blood glucose, to do so, the electrochemical sensor has to include enzyme in its channel. In order to save space for test strip, the electrode set for detecting the blood glucose concentration is disposed between the first conducting portion and the second conducting portion, when the liquid sample flows into the channel, the electrode set begins dection at the same time. In other words, the reaction of the enzyme and the detection of the flow time happen in the same channel and could easily interfere with each other; besides, the enzyme disposed on the electrode set also comprises mixtures such as polymeric binders, stabilizers, buffers, surfactants, which could cause the fluidity of the liquid sample to change and often lead to differences in flow time detection. Besides, since enzyme is provided for reacting with the analyte of the liquid sample to detect the flow time, the flow time signal will not be obtained until the blood samples reacted with the enzyme, otherwise a weak signal or a delayed signal will be detected. Therefore, the prior art technique cannot provide stable dectection results and often fails to reproduce itself.

US patent No.7258769 uses enzymes to react with blood samples to detect the fluidity of blood and the concentration of blood glucose, when enzymes are added to the test strip, the following reactions would occur:

Glucose + Gox-FAD → Gluconic acid + Gox-FADH₂ Gox-FADH₂ + Mox → Gox-FAD + Mred

In the reaction formula, Gox stands for Glucose Oxidase, which reacts with blood glucose to transform into a reduced state, and then the reduced Gox reacts with electron transfer mediators to let the electron transfer mediators transform into a reduced state. Afterwards, the reduced electron transfer mediators would spead to the surface of the electrode and are oxidized by the anode, thereby generating a current for obtaining the concentration of blood glucose. When performing the fluidity detection, it is necessary to wait for blood glucose to react with enzymes to generate a detectable signal, however, by that time the blood may has already flowed through the electrode, so the generated signal does not reflect the real fluidity. Therefore, enzymes disposed in the channel can be used for detecting blood glucose but not for determining the flow time. Since the detection signal can only be generated after the blood sample reacts with enzymes, there will be a time difference between the actual fluidity and the measured fluidity.

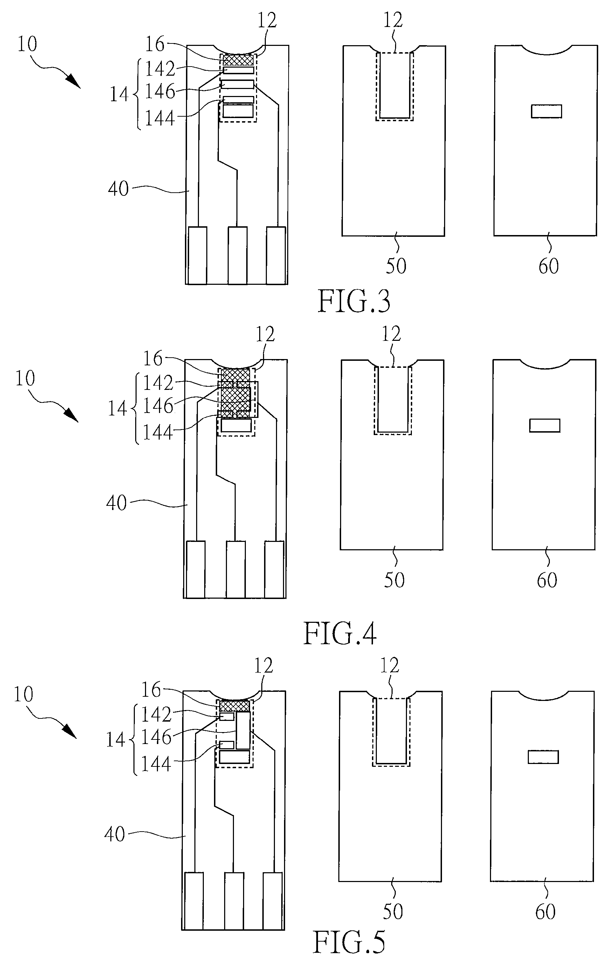

US Patent No.8080153 proposed a method and a system of determining a hematocrit-corrected concentration value of an analyte in a sample. The method comprising: using three reference electrodes with a working electrode in a sampling area to determine a fill time of the sample on the test strip, using enzymes in the sampling area to detect a concentration of the analyte, and then calculating a hematocrit-corrected concentration of the analyte using an empirical formula with the fill time. As shown in FIG.4 of this patent, it is clear that when the hematocrit increases, the fill time values tend to scatter, which implies that the patent does not do well in reproducing itself. As showin in FIG.5, when the hematocrit increases, the concentration of blood glucose reduces, and the number of red blood cells increases. Red blood cells tend to affect the reaction between electron transfer mediators and blood glucose; besides, blood plasma could affect the diffusion of electron transfer mediators as well, so the concentration of blood glucose could be lower than expected. In FIG.4, when the fill time is 0.8, it is difficult to determine the hematocrit (which could be 55% or 65%), which in turn would affect the value used to compensate the blood glucose; in other words, this patent could obtain an undesired corrected concentration of the analyte. Generally a male adult has a hematocrit value of between 39 to 50%, while a female adult could has a hematocrit value of between 36 to 45%. A diabetic often suffers from other complications such as high blood pressure, anemia or other heart disease, so the hematocrit of the diabetic could easily become abnormal. When the hematocrit exceeds the normal range, the concentration of the blood glucose could have apparent deviations and needs to be corrected to avoid erroneous judgement and even putting life in danger.

Since the prior art techniques cannot precisely obtain blood viscosity within a short amount of measurement time. The present invention discloses a test strip and a detecting device to solve the problems present in the prior art techniques.

### SUMMARY OF THE INVENTION

It is an object of the present invention to provide a test strip working with an electrochemical instrument to use a redox reagent to detect the flow time of a specimen and provide a sufficient impulse signal for the electrochemical instrument to accurately detect a viscosity of the specimen. In order to achieve the above object, the present invention provides a test strip, the test strip comprising: a specimen path, an electrode set, and a redox reagent, the specimen path comprising an inlet end and a discharge end; the electrode set having at least a portion thereof disposed in the specimen path, the electrode set at least comprising a first electrode, a second electrode and a reference electrode; a redox reagent disposed in the specimen path, the redox reagent at least comprising a redox pair; when the specimen enters the first specimen path, the redox pair dissolves and generates an electrochemical redox reaction for generating a first impulse signal when the specimen is in contact with the first electrode and the reference electrode and generating a second impulse signal when the specimen is in contact with the second electrode and the reference electrode, thereby obtaining a flow time of the specimen according to the first impulse signal and second impulse signal, and then obtaining a viscosity of the specimen according to the flow time.

It is another object of the present invention to provide a detecting device for detecting a specimen; the detecting device can detect the flow time of the specimen and a concentration of the analyte, and uses the flow time to correct the concentration of the analyte. In order to achieve the object, the present invention provides a detecting device comprising a test strip amd an electrochemical instrument. The test strip comprises a first specimen path, a first electrode set, a redox reagent, a second specimen path, a second electrode set, and a reaction reagent; the first specimen path comprising an inlet end and a discharge end; at least a portion of the first electrode set is disposed in the first specimen path, the first electrode set at least comprises a first electrode, a second electrode, and a first reference electrode; a redox reagent disposed in the first specimen path, the redox reagent at least comprising a redox pair; when the specimen enters the first specimen path, the redox pair dissolves and generates an electrochemical redox reaction for generating a first impulse signal when the specimen is in contact with the first electrode and the first reference electrode and generating a second impulse signal when the specimen is in contact with the second electrode and the first reference electrode, thereby obtaining a flow time of the specimen according to the first impulse signal and second impulse signal; a second specimen path comprising an inlet end and a discharge end; a second electrode set disposed in the second specimen path, the second electrode set at least comprising a working electrode, a detector electrode, and a second reference electrode; and a reaction reagent disposed in the second specimen path, the reaction reagent at least comprising an enzyme for detecting a concentration of an analyte of the specimen; and an electrochemical instrument electrically connected with the test strip and used for obtaining the flow time and the concentration of the analyte, the electrochemical instrument using the flow time to correct the concentration of the analyte.

### BRIEF DESCRIPTION OF THE DRAWINGS

FIG. 1 illustrates a view of a test strip working with an electrochemical instrument to detect a specimen;
FIG.2 to FIG.14 illustrate various structures of the test strip according to an embodiment of the present invention;
FIG.15 illustrates a view of using the detecting device to detect according to an enbodiment of the present invention;
FIG.16 to FIG.59B illustrate various structures of the test strip of the detecting device according to an embodiment of the present invention;
FIG.60A to FIG.62H illustrate various structures of a first specimen path in series with a second specimen path of the test strip of the detecting device according to an embodiment of the present invention;
FIG.63A to FIG.74B illustrate various structures of a time detector electrode disposed in the test strip of the detecting device according to an embodiment of the present invention;
FIG.75 to FIG.86 illustrate flow charts of a detection method according an embodiment of the present invention; and
FIG.87A to 87B shows the results of using venous bloods of different hematocrits as different viscosity conditions versus blood glucose values.

### DETAILED DESCRIPTION OF THE PREFERRED EMBODIMENT

The advantages and innovative features of the invention will become more apparent from the following detailed description when taken in conjunction with the accompanying drawings.

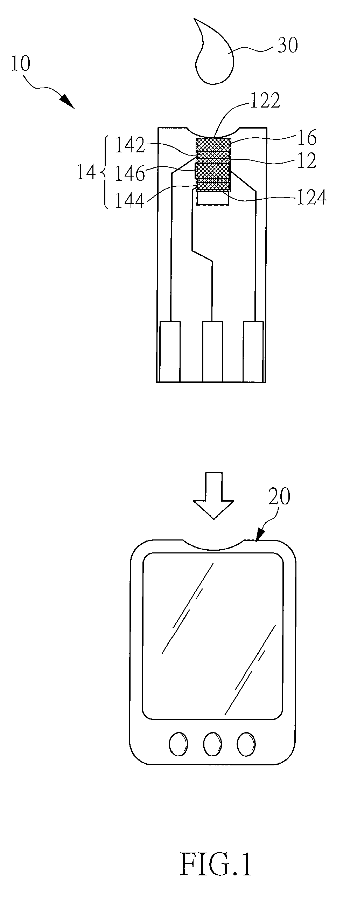

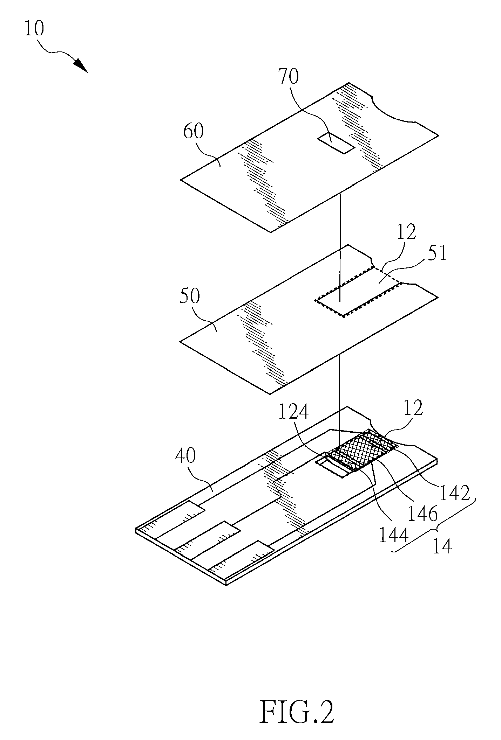

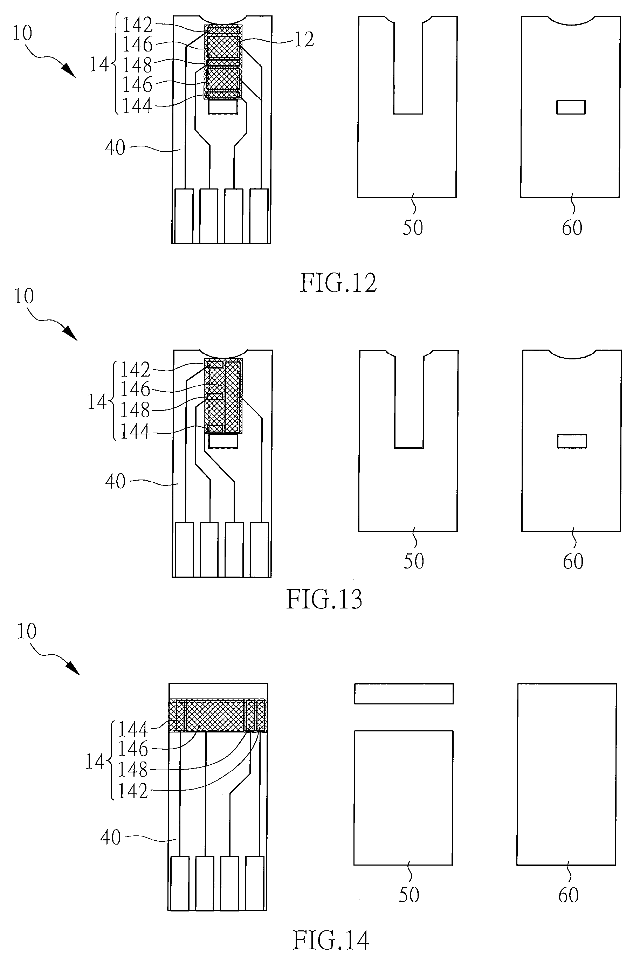

First, the present invention provides a test strip for detecting a flow time of the specimen and using the flow time to calculate the viscosity of the specimen; so the test strip can work with the electrochemical instrument to form a viscosity detecting device. Please refer to the test strip illustrated in FIG. 1 to FIG.14. FIG. 1 illustrates a view of a test strip working with an electrochemical instrument to detect a specimen; and FIG.2 to FIG.14 illustrate various structures of the test strip according to an embodiment of the present invention.

First, please refer to FIG.1, according to an embodiment of the present invention, the present invention provides a test strip 10 inserted in an electrochemical instrument 20 to work with the electrochemical instrument 20 to detect a specimen 30. In an embodiment of the present invention, the specimen 30 can be blood, urine, saliva, or the like.

In an embodiment of the present invention, the test strip 10 comprises a specimen path 12, an electrode set 14, and a redox reagent 16. The specimen path 12 comprises an inlet end 122 and a discharge end 124; at least a portion of the electrode set 14 is disposed in the specimen path 12; the electrode set 14 comprises a first electrode 142, a second electrode 144, and a reference electrode 146; the redox reagent 16 is disposed in the specimen path 12, the redox reagent 16 at least comprises a redox pair comprising an oxidizer and a reducer, wherein the oxidizer and reducer would have their oxidation numbers shifted with respect to each other after the chemical reaction.

In an embodiment of the present invention, the redox pair comprises potassium ferricyanide and potassium ferrocyanide, however, the redox pair can comprise any other suitable materials such as hexaamineruthenium (III) chloride, potassium ferricyanide, potassium ferrocyanide, dimethylferrocene, ferricinium, ferocene-monocarboxylic acid, 7,7,8,8-tetracyanoquinodimethane, tetrathiafulvalene, nickelocene, N-methylacidinium, tetrathiatetracene, N-methylphenazinium, hydroquinone, 3-dimethylaminobenzoic acid, 3-methyl-2-benzothiozolinone hydrazone, 2-methoxy-4-allylphenol, 4-aminoantipyrin, dimethylaniline, 4-aminoantipyrene, 4-methoxynaphthol, 3,3',5, 5'-tetramethylbenzidine, 2,2-azino-di-[3-ethylbenzthiazoline sulfonate], o-dianisidine, o-toluidine, 2,4-dichloro phenol, 4-aminophenazone, benzidine and Prussian blue.

It is noted that other materials other than those described above can be used as oxidizers and reducers. Besides, the redox reagent 16A can further comprises surfactants and buffers. Preferred oxidizers and reducers have low redox voltage levels. They can reduce supplied power and save cost, and also eliminate the possibilities of triggering other redox reactions.

As shown in FIG.1, the test strip 10 can work with the electrochemical instrument 20 to detect the specimen 30. The redox reagent 16, at least a portion of the first electrode 142, the second electrode 144, and the reference electrode 146 are disposed in the specimen path 12; the first electrode 142, the second electrode electrode 144, and the reference electrode 146 are separated from one another. Therefore, before the specimen 30 is detected, the second electrode electrode 144, and the reference electrode 146 are electrically isolated from one another.

As shown in FIG.1, taking account of air pressure and in order to let the specimen 30 flow in the specimen path 12 without being blocked by air, the discharge end 124 is disposed in the specimen path 12 of the test strip 10. When the specimen 30 is drawn into the specimen path 12, air existed in front of the specimen 30 can be discharged to facilitate flowing of the specimen 30. By the design of the discharge end 124, when the specimen 30 enters the specimen path 12, it will move towards the discharge end 124. When flowing in the specimen path 12, the specimen 30 will be in contact with the redox reagent 16 first, and then in the order of the first electrode 142, the reference electrode 146, and the second electrode 144 in the last. Therefore, when the specimen 30 enters the specimen path 12, it makes contact with the redox pair, wherein the redox pair dissolves and generates an electrochemical redox reaction under the voltage applied by the electrochemical instrument 20. As the specimen 30 flows along the specimen path 12 to makes contact with the first electrode 142 and the reference electrode 146, it forms a conducting loop with the first electrode 142 and the reference electrode 146 to generate a first impulse signal; consequently, as the specimen 30 flows along the specimen path 12 to makes contact with the reference electrode 146 and the second electrode 144, it forms another conducting loop with the reference electrode 146 and the second electrode 144 to generate the second impulse signal.

Thereafter, the electrochemical instrument 20 can calculate a flow time of the specimen 30 according to the first impulse signal and the second impulse signal and then obtain a viscosity of the specimen 30 based on the flow time. Since the distances between the first electrode 142, the second electrode 144, and the reference electrode 146 in the specimen path 12 are predetermined, the flow time can be obtained by using the distances and a time difference between the first impulse signal and the second impulse signal, and the viscosity of the specimen 30 can be obtained as well. Since the calculation of the viscosity based on the flow time is known in the art, it will not be further described.

According to an embodiment of the present invention, the redox pair in the redox reagent 16 does not generate a redox reaction before making contact with the specimen 30, the redox pair generates the redox reaction only after it dissolves in the specimen 30 and flows through the electrode set 14 provided voltage by the electrochemical instrument 20. At this time the specimen 30 is used as a solvent and does not participate in the reaction, while the reactivity of the specimen 30 will be improved by electrons generated by the oxidizers and reducers in the redox reaction. The present invention provides the redox reagent 16 to ensure the accuracy of the first impulse signal and the second impulse signal, thereby obtaining the viscosity of the specimen 30.

The present invention provides the redox reagent 16 to improve the reactivity of the specimen 30 with fast and immediate effects. When the redox reagent is dissolved in the specimen 30, it provides sufficient reactants to generate a clear impulse signal, thereby reflecting a real status of the fluidity of the specimen 30 in the test strip 10.

As shown in FIG.1, in an embodiment of the present invention, the first electrode 142 in the specimen path 12 is disposed near the inlet end 122 of the specimen path 12, the second electrode 144 is disposed near the discharge end 124 of the specimen path 12, and the reference electrode 146 is disposed between the first electrode 142 and the second electrode 144; however, the electrode set 14 can be configured differently, which will be described later.

As shown in FIG.1, in an embodiment of the present invention, the redox reagent 16 covers at least a portion of the electrode set 14, the redox reagent 16 can be disposed elsewhere as long as it can be dissolved in the specimen 30 when the specimen 30 is in contact with the electrode set 14. The redox reagent 16 can be configured differently, which will be described later.

As shown in FIG.1, in an embodiment of the present invention, the inlet end 122 of the specimen path 12 is disposed in front of the test strip 10. However, the inlet end 122 of the specimen path 12 can be disposed at a side of the test strip 10 or any suitable places, which will be described later.

As shown in FIG.1, in an embodiment of the present invention, the electrode set 14 comprises the first electrode 142, the second electrode 144 and the reference electrode 146; however, the electrode set 14 can comprises other additional electrodes to increase the accuracy in calculating the flow time, which will be described later.

Please refer to FIG.2 to FIG. 14 for various structures of the test strip according to an embodiment of the present invention; in which configurations for disposing various electrode sets, redox reagents, and specimen paths are illustrated.

Please refer to FIG.2 for a structural view of test strip according to an embodiment of the present invention. As shown in FIG.2, the test strip 10 comprises a substrate 40, a spacer layer 50, and a cover layer 60. The electrode set 14 is disposed on the substrate 40; the spacer layer 50 covers the substrate 40 and exposes a portion of the electrode set 14; and the cover layer 60 covers the spacer layer 50 to form the specimen path 12.

As shown in FIG.3, in an embodiment of the present invention, a breach 51 is form in the spacer layer 50 to correspond to the shape of the specimen path 12, thereby allowing the specimen 30 to flow in the specimen path 12. Furthermore, the test strip 10 comprises a through hole 70 penetrating through the substrate 40, the spacer layer 50, and the cover layer 60 to communicate with the discharge end 124 of the specimen path 12, thereby increasing the area for discharging air and facilitating flowing of the specimen 30 in the specimen path 12. The through hole is disposed to stop the specimen 30 at the discharge end, so the specimen 30 will flow in the capillary and will not be drawn by the cover layer 60 or the substrate 40 to leave the capillary. It is noted that the present invention can have discharge holes disposed on the cover layer 60 or the substrate 40 respectively without using the through hole 70 and can still serve the purpose.

As shown in FIG.3, in an embodiment of the present invention, the redox reagent 16 is disposed in front of the electrode set 14. When the specimen 30 enters the specimen path 12, the specimen 30 is in contact with the redox pair in the redox reagent 16; then the specimen 30 carries the redox reagent 16, dissolves the redox pair and makes contact with the electrode set 14.

As shown in FIG.4, in an embodiment of the present invention, the first electrode 142 in the specimen path 12 is disposed near the inlet end 122 of the specimen path 12, the second electrode 144 is disposed near the discharge end 124 of the specimen path 12, and the reference electrode 146 is formed in a fork shape having two ends disposed near the first electrode 142 and the second electrode 144 respectively. In other words, one end of the fork-like reference electrode 146 is near the inlet end 122 of the specimen path 12, while the other end of the fork-like reference electrode 146 is near the discharge end 124 of the specimen path 12.

As shown in FIG.5, in an embodiment of the present invention, the present invention can have both the configuration of the redox reagent 16 in FIG. and the configuration of the reference electrode 146 in FIG.4. That is, the redox reagent 16 is disposed in front of the electrode set 14 and disposed in the specimen path 12; the reference electrode 146 is formed in a strip shape, two ends of the reference electrode 146 are disposed near the first electrode 142 and the second electrode 144 respectively.

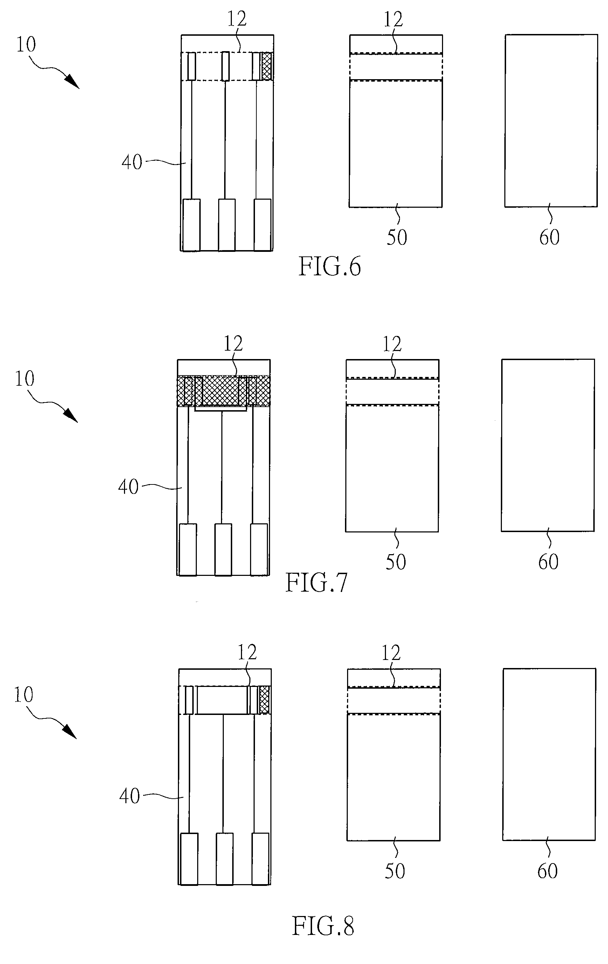

As shown in FIG.6 to FIG.8, in an embodiment of the present invention, while the inlet end 122 of the specimen path12 shown in FIG.3 to FIG.5 is disposed at a front end of the test strip 10, the inlet end 122 of the specimen path12 can be disposed at a side of the test strip 10. In the embodiment shown in FIG.3 to FIG.5, the specimen 30 is drawn from the front end of the test strip 10, while in the embodiment shown in FIG.6 to FIG.8, the specimen 30 is drawn from the side, but the specimen 30 still flows in the specimen path 12 and makes contact with the redox reagent 16 and the electrode set 14; therefore, the basic principle and technical feature do not change.

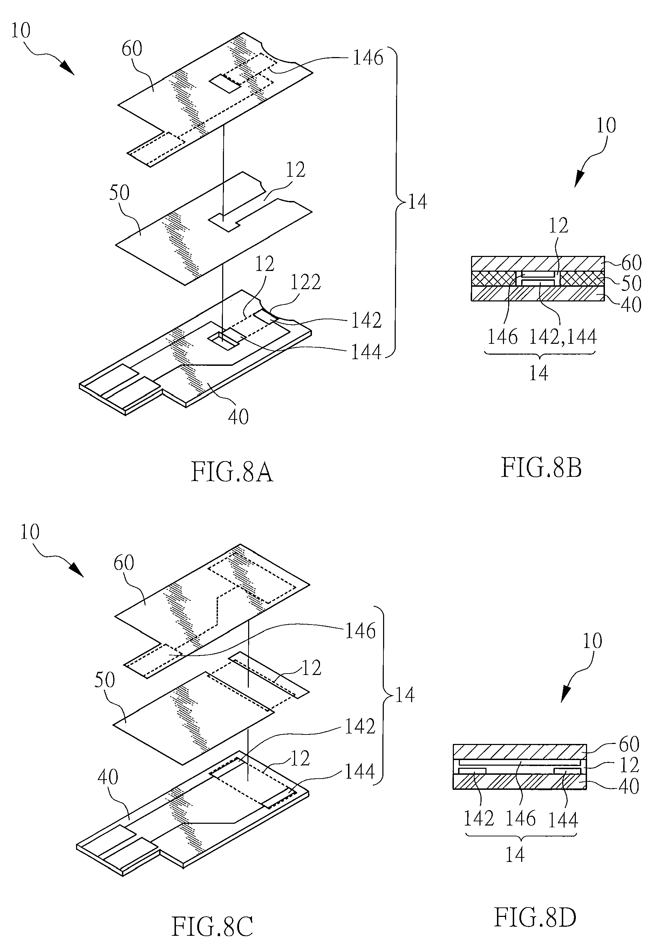

Additionally, as shown in FIG.8A to FIG.8D, the electrode set is formed in a stack, wherein the reference electrode 146 is on a plane different from the plane on where the first electrode 142 and the second electrode 144 are disposed. FIG.8A and FIG.8C are explosive views of the stack-like electrode set of the test strip according to an embodiment of the present invention; FIG.8B and FIG.8D illustrate the test strip as a whole. As show in FIG.8A to FIG.8D, in an embodiment of the present invention, the first electrode 142 and the second electrode 144 are disposed on the substrate 40; the spacer layer 50 covers the substrate 40 and exposes a portion of the first electrode 142 and the second electrode 144; the cover layer 60 covers the spacer layer 50 and has the reference electrode 146 disposed on a lower surface of the cover layer 60, thereby forming the electrode set 14 in a stack and forms the specimen path 12 as a whole. It is noted that when the electrode set 14 is disposed in a stack, the inlet end 122 of the specimen path 12 can be disposed at a front end of the test strip 10 (as shown in FIG.8A and FIG.8B), or the inlet end 122 of the specimen path 12 can be disposed at a side of the test strip 10 (as shown in FIG.8C and FIG.8D).

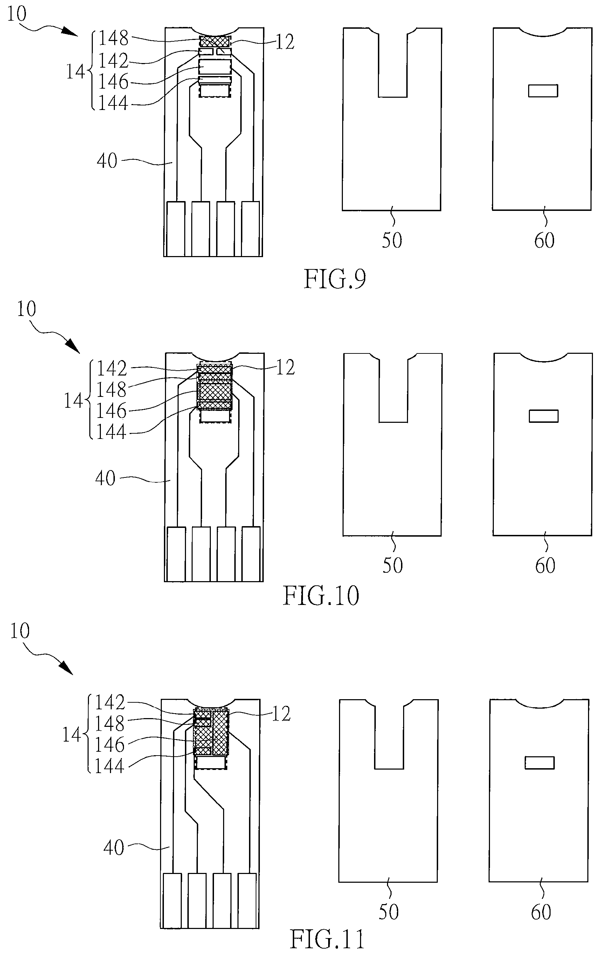

As shown in FIG.9 to FIG.14, in an embodiment of the present invention, the electrode set 14 of the test strip 10 further comprises a third electrode 148. When the specimen 30 flows through the third electrode 148 and the reference electrode 146, a third impulse signal is generated for the detecting device to calculate the flow time of the specimen 30 according to the first impulse signal, the second impulse signal, and the third impulse signal, thereby obtaining the viscosity of the specimen 30 according to the flow time. As shown in FIG.9, the third electrode 148 in the specimen path 12 is disposed near the first electrode 142; alternatively, as shown in FIG. 10 to FIG. 14, the third electrode 148 in the specimen path 12 is disposed between the first electrode 142 and the second electrode 144. With the third electrode 148, at least two sets of flow time values can be obtained to verify the recorded flow time, if one flow time value is much different from another flow time value, then an error alert is issued to a user. Furthermore, in the embodiment illustrated in FIG.9 to FIG. 14, there can be different configurations for the electrode set, the redox reagent, and the specimen path.

Additionally, the width of the specimen path 12 is varied. When the width of the specimen path 12 increases, the flow rate of the specimen 30 could be too fast, and the fluidity of the specimen 30 could be easily affected by the user (such as shaking, swaying or flipping) or the placement (such as inserted with different orientations) of the test strip 10. However, if the width of the specimen path 12 is too narrow, then the flow time of the specimen 30 could be prolonged, making it difficult for the specimen 30 to enter the specimen path 12. Therefore, in an embodiment of the present invention, when the specimen 30 is blood, the width of the specimen path 12 is preferably to be 0.2 to 2 mm, the length is preferably to be 5 to 15 mm, and the volume is 0.1 to 1 micro liter, this configuration can maintain fine fluidity of the specimen 30 without being affected by gravity, and also provides quick and convenient features to the test strip 10.

As above, when the test strip 10 is inserted into the electrochemical instrument 20, the electrochemical instrument 20 provides a voltage to the first electrode 142, the second electrode 144, and the reference electrode 146. When the specimen 30 dissolved with the redox reagent passes through each electrode in the specimen path 12, the redox reaction is generated. The electrochemical instrument 20 measures and records all impulse signals obtained from conducting loops and uses the time differences between the impulse signals to calculate the viscosity of the specimen 30.

In an embodiment of the present invention, the test strip 10 is connected with the electrochemical instrument 20 through a slot of the electrochemical instrument 20, so the user can just insert one end of the test strip 10 that comprises the exposed electrodes to the slot. Besides, the electrode set 14can be made of any conducting materials such as Pd, Au, Pt, Ag, Ir, C, Indium Tin Oxide, Indium Zinc Oxide, Cu, Al, Ga, Fe, Hg, Ta, Ti, Zr, Ni, Os, Re, Rh, Pd, organic metal and other conductive materials. Furthermore, the electrode set 14 can be formed by sputtering, vapor deposition, screen printing or any other suitable manufacturing methods. For example, one or more electrode can be made at least partly by sputtering, deposition, supersonic vaporization, pressurized vaporization, direct writing, mask etching, or laser ablation.

The present invention also provides a detecting device for detecting a specimen, wherein the detecting device detects the flow time of the specimen and the concentration of the analyte, in an embodiment of the present invention, the detecting device can be used as a blood glucose detecting device.

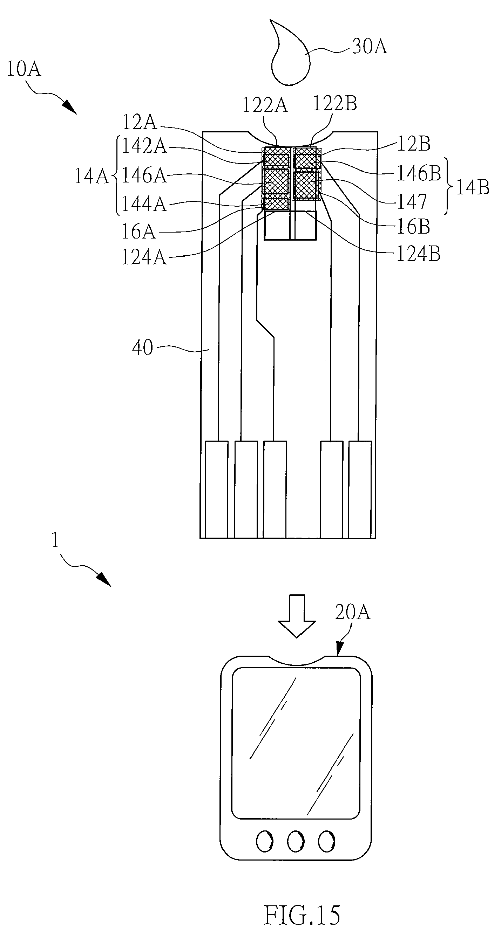

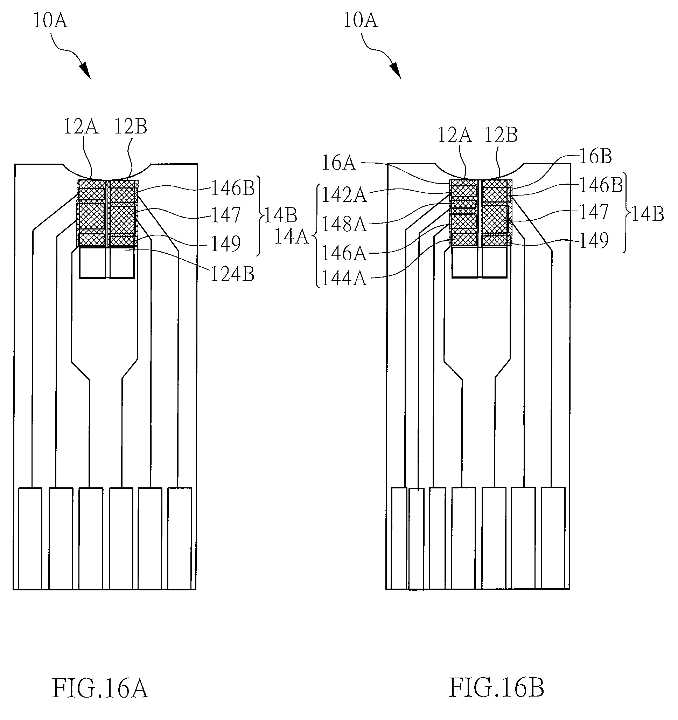

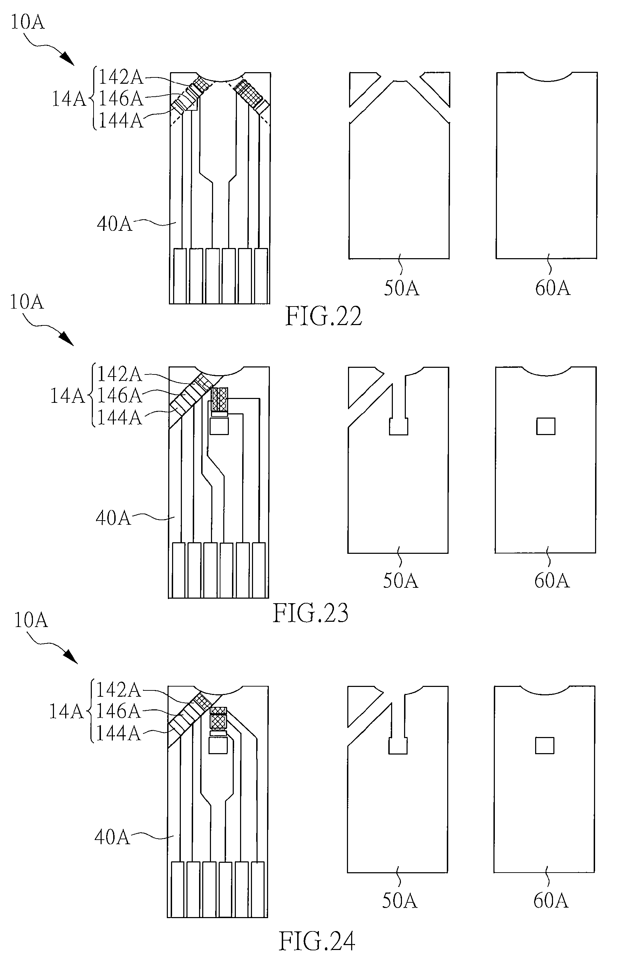

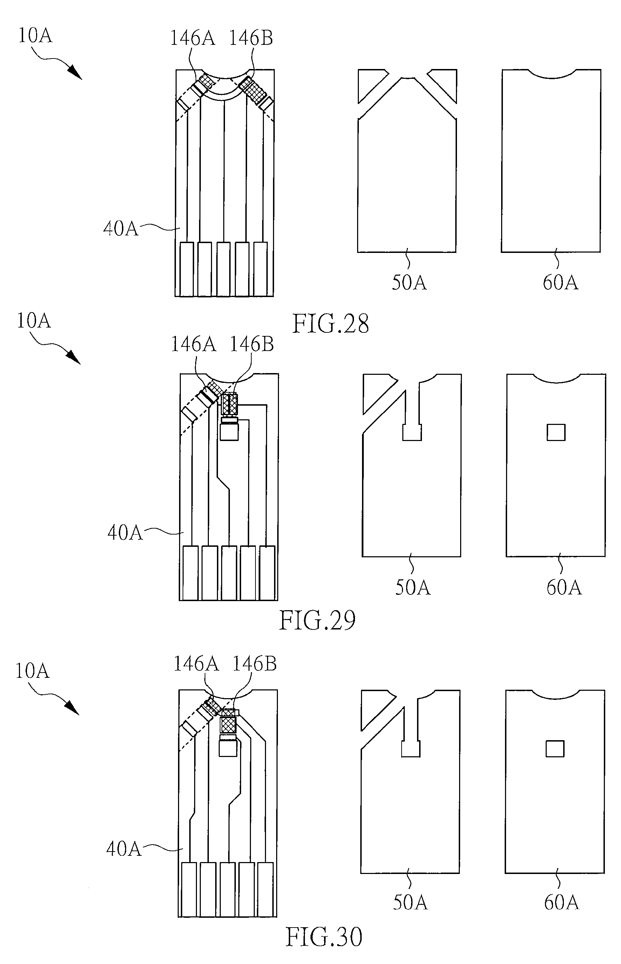

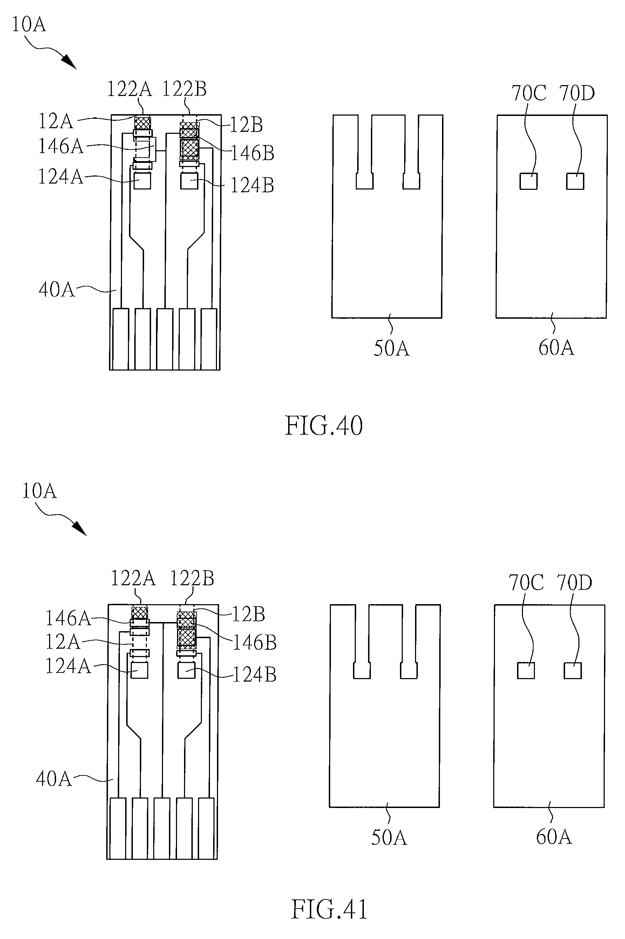

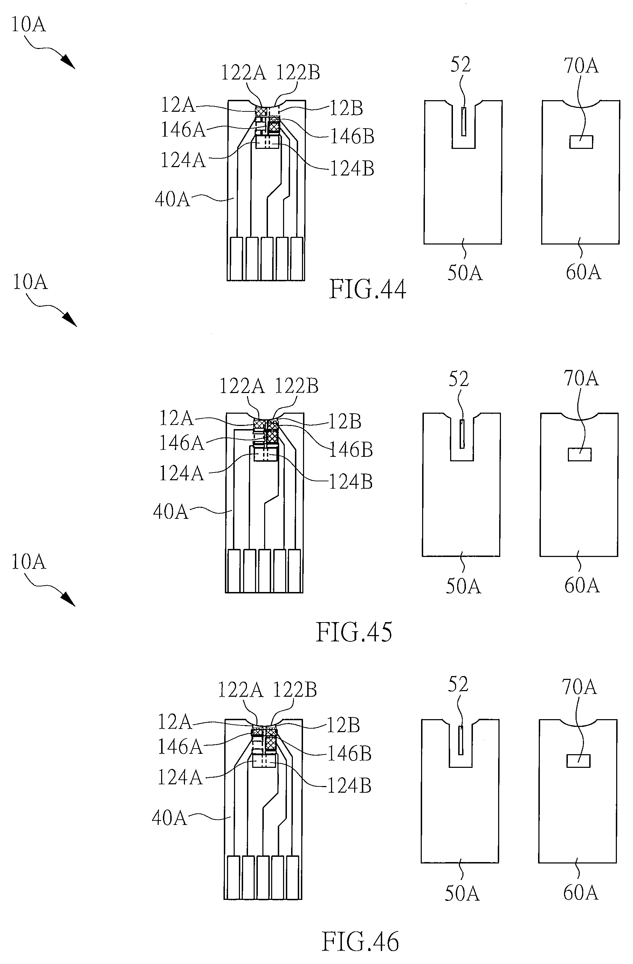

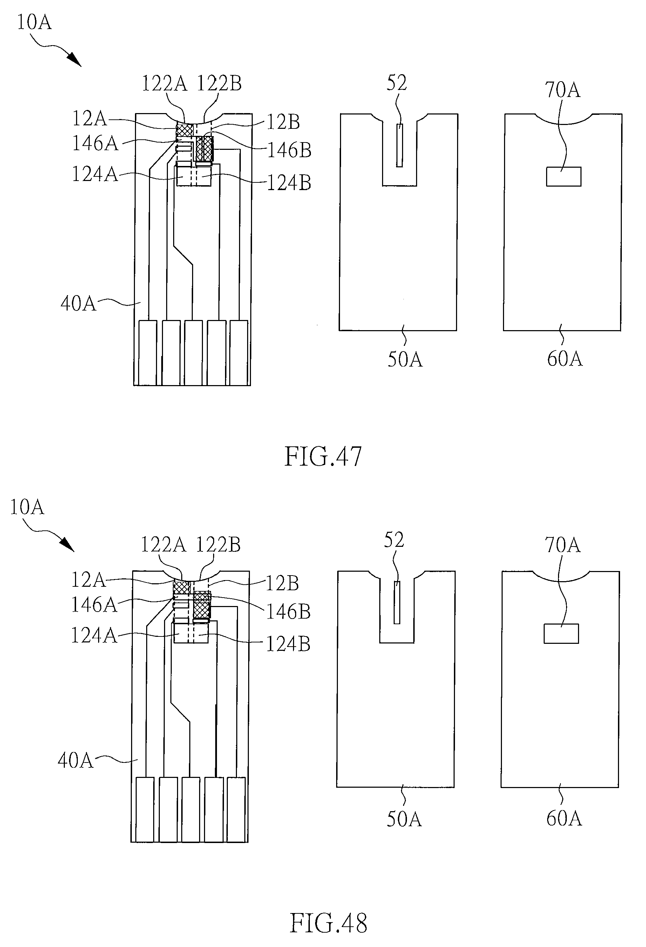

Please refer to FIG.15 to FIG.59B for the detecting device of the present invention. FIG.15 illustrates a view of using the detecting device to detect according to an enbodiment of the present invention; FIG.16 to FIG.59B illustrate various structures of the test strip of the detecting device according to an embodiment of the present invention.

First, please refer to FIG.15, according to an embodiment of the present invention, the present invention provides a detecting device 1 comprising a test strip 10A and an electrochemical instrument 20A. The test strip 10A is inserted into the electrochemical instrument 20A and works with the electrochemical instrument 20A to detect the specimen 30A. In an embodiment of the present invention, the specimen 30A can be blood, urine, saliva, or the like.

As shown in FIG.15, according to an embodiment of the present invention, the present invention provides a test strip 10A comprising: a first specimen path 12A, a first electrode set 14A, a redox reagent 16A, a second specimen path 12B, a second electrode set 14B, and a reaction reagent 16B.

The first specimen path 12A comprises an inlet end 122A and a discharge end 124A; at least a portion of the first electrode set 14A is disposed in the first specimen path 12A; the first electrode set 14A comprises a first electrode 142A, a second electrode 144A, and a reference electrode 146A; the redox reagent 16A is disposed in the first specimen path 12A, the redox reagent 16A at least comprises a redox pair comprising an oxidizer and a reducer, wherein the oxidizer and reducer would have their oxidation numbers shifted with respect to each other after the chemical reaction.

In an embodiment of the present invention, the redox pair comprises potassium ferricyanide and potassium ferrocyanide, however, the redox pair can comprise any other suitable materials such as hexaamineruthenium (III) chloride, potassium ferricyanide, potassium ferrocyanide, dimethylferrocene, ferricinium, ferocene-monocarboxylic acid, 7,7,8,8-tetracyanoquinodimethane, tetrathiafulvalene, nickelocene, N-methylacidinium, tetrathiatetracene, N-methylphenazinium, hydroquinone, 3-dimethylaminobenzoic acid, 3-methyl-2-benzothiozolinone hydrazone, 2-methoxy-4-allylphenol, 4-aminoantipyrin, dimethylaniline, 4-aminoantipyrene, 4-methoxynaphthol, 3,3',5, 5'-tetramethylbenzidine, 2,2-azino-di-[3-ethylbenzthiazoline sulfonate], o-dianisidine, o-toluidine, 2,4-dichloro phenol, 4-aminophenazone, benzidine and Prussian blue. It is noted that other materials other than those described above can be used as oxidizers and reducers. Besides, the redox reagent 16A can further comprises surfactants and buffers. Preferred oxidizers and reducers have low redox voltage levels. They can reduce supplied power and save cost, and also eliminate the possibilities of triggering other redox reactions.

The second specimen path 12B comprises an inlet end 122B and a discharge end 124B; the second electrode set 14B has at least a portion disposed in the second specimen path 12B and at least comprises a working electrode 147 and a second reference electrode 146B; the reaction reagent 16B is disposed in the second specimen path 12B, the reaction reagent 16B at least comprises a specific enzyme for detecting the concentration of the analyte of the specimen 30A. In an embodiment of the present invention, the reaction reagent 16B also comprises polymeric binders, buffers, surfactants, and electron transfer mediators. In an embodiment of the present invention, the analyte can be blood glucose, lipid, cholestrol, uric acid, alcohol, triglycerides, ketone body, creatinine, lactic acid, haem, or the like.

It is noted that since enzymes could affect the accuray of fluidity test, therefore, in an embodiment of the present invention, the redox reagent 16A of the present invention does not comprise any enzyme to avoid affecting the fluidity test in the first specimen path 12A.

As shown in FIG.15, the test strip 10A can work with the electrochemical instrument 20A to detect the specimen 30A. The redox reagent 16A, at least a portion of the first electrode 142A, the second electrode 144A, and the first reference electrode 146A are disposed in the first specimen path 12A; the first electrode 142A, the second electrode 144A, and the first reference electrode 146A are separated from one another. Besides, the reaction reagent 16B and a least a portion of the working electrode 147 and the second reference electrode 146B are disposed in the second specimen path 12B; wherein the working electrode 147 and the second reference electrode 146B are separated from each other.

Therefore, before the specimen 30A is detected, the first electrode 142A, the second electrode 144A, and the first reference electrode 146A are electrically isolated from one another; the working electrode 147 and the second reference electrode 146B are electrically isolated from each other. As shown in FIG.15, when the specimen 30A enter the first specimen path 12A, the redox pair dissolves and generates an electrochemical redox reaction under the voltage applied by the electrochemical instrument 20A. As the specimen 30A flows along the first specimen path 12A to make contact with the first electrode 142A and the first reference electrode 146A, it generates a first impulse signal; consequently, as the specimen 30 makes contact with the first reference electrode 146A and the second electrode 144A, it generates the second impulse signal. The first impulse signal and the second impulse signal are used for calculating a flow time of the specimen 30A.

Futhermore, when the specimen 30A enters the second specimen path 12B, it reacts with enzymes of the reaction reagent 16B, so when the specimen 30A makes contact with the working electrode 147 and the second reference electrode 146B, a response signal is generated for calculating the concentration of the analyte of the specimen 30A.

As shown in FIG. 15, taking account of air pressure and in order to let the specimen 30A flow in the first specimen path 12A and/or the second specimen path 12B without being blocked by air, test strip 10A comprises the discharge end 124A and 124B. When the specimen 30A is drawn into the first specimen path 12A and/or second specimen path 12B, air existed in front of the specimen 30A can be discharged to facilitate flowing of the specimen 30A. By the design of the discharge end 124A and 124B, when the specimen 30A enters the first specimen path 12A and/or the second specimen path 12B, it will move towards the discharge end 124A and/or 124B. When flowing in the first specimen path 12A, the specimen 30A will be in contact with the redox reagent 16A first, and then in the order of the first electrode 142A, the first reference electrode 146A, and the second electrode 144A in the last. Therefore, when the specimen 30A enters the first specimen path 12A, it first makes contact with the redox pair in the redox reagent 16A, wherein the redox pair dissolves and generates an electrochemical redox reaction under the voltage applied by the electrochemical instrument 20A. As the specimen 30A flows along the first specimen path 12A to makes contact with the first electrode 142A and the first reference electrode 146A, it forms a conducting loop with the first electrode 142A and the first reference electrode 146A to generate a first impulse signal; consequently, as the specimen 30A flows along the first specimen path 12A to make contact with the first reference electrode 146A and the second electrode 144A, it forms another conducting loop with the first reference electrode 146A and the second electrode 144A to generate the second impulse signal.

Thereafter, the electrochemical instrument 20A can calculate a flow time of the specimen 30A according to the first impulse signal and the second impulse signal and then obtain a viscosity of the specimen 30A based on the flow time. Since the distances between the first electrode 142A, the second electrode 144A, and the first reference electrode 146A in the first specimen path 12A are predetermined, the flow time can be obtained by using the distances and a time difference between the first impulse signal and the second impulse signal, and the viscosity of the specimen 30A can be obtained as well. Since the calculation of the viscosity based on the flow time is known in the art, it will not be further described.

According to an embodiment of the present invention, the redox pair in the redox reagent 16A does not generate a redox reaction before making contact with the specimen 30A, the redox pair generates the redox reaction only after it dissolves in the specimen 30A and flows through the first electrode set 14A provided voltage by the electrochemical instrument 20A. At this time the specimen 30A is used as a solvent and does not participate in the reaction, while the reactivity of the specimen 30A will be improved by electrons generated by the oxidizers and reducers in the redox reaction. The present invention provides the redox reagent 16A to ensure the accuracy of the first impulse signal and the second impulse signal, thereby obtaining the flow time, and even the viscosity of the specimen 30A.

As shown in FIG.15, the specimen 30A flows both in the first specimen path 12A and the second specimen path 12B. When the specimen 30A flows in the second specimen path 12B, it will first make contact with a specific enzyme in the reaction reagent 16B and react with the analyte of the specimen 30A; then the specimen 30A will be in contact with the second reference electrode 146B and the working electrode 147 sequentially to obtain a concentration of the analyte of the specimen 30A.

As shown in FIG.15, the present invention disposes the redox reagent 16A comprising a redox pair in the first specimen path 12A and disposes the reaction reagent 16B in the second specimen path 12B to obtain the flow time and the analyte concentration of the specimen 30A. Since different reagents are disposed in the first specimen path 12A and the second specimen path 12B respectively for doing different detecting jobs, so it is possible to detect the flow time and the analyte concentration sepearated without interference. Besides, since the redox reagent has nothing to do with the fluidity of the specimen 30A, when the specimen 30A flows through the paths, the redox reagent can be immediately dissolved, thereby improving the detection of the flow time of the specimen 30A and obtaining the precise viscosity of the specimen 30A. The present invention can also use the accurate flow time to correct the concentration of the analyte of the specimen 30A and to obtain an accurate concentration of the analyte.

The present invention uses the redox reagent 16A to improve the reactivity of the specimen 30A with fast and immediate effects; therefore, the result can be obtained before the specimen 30A reacts with enzymes. When the redox reagent is dissolved in the specimen 30, it provides sufficient reactants to generate a clear impulse signal, thereby reflecting a real status of the fluidity of the specimen 30A in the test strip 10A.

In an embodiment of the present invention, the specimen 30A is blood, and the concentration of the analyte refers to the concentration of blood glucose. Since blood is a mixture of many physiological substances, when using an electrochemical method to obtain the concentration of an analyte of blood, it is necessary to go through corrections and compensation steps to obtain an accurate result. For example, the concentration of blood glucose varies with different hematocrits. While the normal value of hematocrit is between 35 to 55%, the hematocrit value for anemia patients would be lower, and the hematocrit value for babies would be little higher, making it difficult to judge whether the hematocrit value is within a normal range. Besides, US standards for clinical diagnosis center listed sixteen electrochemical interference substances, which include: paracetamol, Vitamin C, salicylic acid, tolbutamide, tetracycline, tolinase, dopamine, bilirubin, ephedrine, cholestrol, Ibuprofen, creatinine, L-dopa, triglycerides, methyldopa, urate.

In the prior art technique, in order to measure the concentration of the analyte in the presence of red blood cells as a interference substance, US patent No. 7407811 discloses a method of measuring an analyte in a biological fluid comprises applying an excitation signal having a DC component and an AC component. The AC responses comprising a phase angle and an admittance value are measured; a corrected DC response is determined using the AC response; and a concentration of the analyte is determined based upon the corrected DC response, thereby obtaining the hematocrit. In an embodiment of the present invention, after the electrochemical instrument 20A obtains the flow time of the specimen 30A, the electrochemical instrument 20A can provide an AC signal to the first electrode set 14A to let the specimen 30A generate a reaction current, which is used for calculating a hematocrit. Afterwards, the hematocrit obtained from the reaction current and the hematocrit obtained from the flow time are compared, if the two values are close, then the concentration of the analyte is corrected and calculated by the flow time to obtain a more accurate concentration of the analyte; if a difference between the two values exceeds a predetermined range, the an error alert is issued to a user. The technique of using AC signals to compensate the concentration of the analyte has been disclosed in US patent No.7407811 and US patent application No.2011/0139634 A1, which are both cited in the present invention.

There are more than one analytes in a blood sample, other substances such as urea, acetaminophen, vitamin C, dihydroxy benzoic acid also exist, and these substances can be oxidizers or reducers. When an electrochemical reaction occurs, these substances would all participate in the electrochemical reaction; therefore, the electrochemical instrument 20A needs to correct or compensate the response signal obtained. In an embodiment of the present invention, after the electrochemical instrument 20A of the present invention obtains the flow time of the specimen 30A, the electrochemical instrument 20A provides a voltage to the first electrode set 14A to let the specimen 30A generate a electrochemical reaction current; this electrochemical reaction current should be the background current of the blood sample or come from interference substances, it is not the reaction current of the concentration of the analyte. Therefore, this electrochemical reaction current could be used to calculate and correct the concentration of the analyte, thereby obtaining a more accurate analyte concentration. In the present invention the voltage used to detect the background current has the same voltage level as that used to detect the concentration of the analyte. Besides, when this electrochemical reaction current is used to compensate the concentration of the analyte, a positive or negative compensation could be achieved. US patent No.7653492 discloses a method of reducing the effect of interference in a specimen when measuring an analyte using an electrochemical sensor. This patent document is cited in the present invention and will not be further described.

As shown in FIG.15, in an embodiment of the present invention, the first electrode 142A in the first specimen path 12A is disposed near the inlet end 122A of the first specimen path 12A, the second electrode 144A is disposed near the discharge end 124A of the first specimen path 12A, and the first reference electrode 146A is disposed between the first electrode 142A and the second electrode 144A; however, the first electrode set 14A can be configured differently, which will be described later.

As shown in FIG.15, in an embodiment of the present invention, the first electrode set 14A and the second electrode set 14B are disposed next to each other, however, the present invention can have other arrangements. The first electrode set 14A and the second electrode set 14B can be disposed in other arrangements, which will be described later.

As shown in FIG.15, in an embodiment of the present invention, the redox reagent 16A covers at least a portion of the first electrode set 14A in the first specimen path 12A; however, in an embodiment of the present invention, the redox reagent 16A can be configured differently as long as the specimen 30A can carry and dissolve the the redox reagent 16A when the specimen 30A is in contact with the the first electrode set 14A. Different configurations of the redox reagent 16A will be described later.

As shown in FIG.15, in an embodiment of the present invention, the reaction reagent 16B covers at least a portion of the second electrode set 14B in the second specimen path 12B; however, in an embodiment of the present invention, the reaction reagent 16B can be configured differently as long as the specimen 30A can react with the enzymes when the specimen 30A is in contact with the the second electrode set 14B. Different configurations of the reaction reagent 16B will be described later.

As shown in FIG. 15, in an embodiment of the present invention, the inlet end 122A of the first specimen path 12A and the inlet end 122B of the second specimen path 12B are disposed at a front end of the test strip 10A. Alternatively, the inlet end 122A of the first specimen path 12A and/or the inlet end 122B of the second specimen path 12B can be disposed at a side of the test strip 10A; other configurations are also possible and will be descried later.

As shown in FIG. 15, in an embodiment of the present invention, the first electrode set 14A comprises the first electrode 142A, the second electrode 144A, and the first reference electrode 146A; however, the first electrode set 14A can also comprise other additional electrodes to improve the accuracy in calculating the flow time. Furthermore, as shown in FIG.15, in an embodiment of the present invention, the second electrode set 14B comprises the working electrode 147 and the second reference electrode 146B; however, the second electrode set 14B of the present invention can comprise other additional electrodes to improve the accuracy in calculating the concentration of the analyte, which will be described later.

As shown in FIG.15, in an embodiment of the present invention, the first specimen path 12A and the second specimen path 12B are disposed in parallel; however, the first specimen path 12A and the second specimen path 12B can be disposed in other arrangements, which will be described later.

Please refer to FIG.16 to FIG.59B for various structures of the test strip of the detecting device according to an embodiment of the present invention; in which various configurations of electrode sets, redox reagents, and specimen paths are illustrated.

As shown in FIG.16A, in an embodiment of the present invention, the second electrode set 14B of the test strip 10A further comprises a detector electrode 149 disposed near the discharge end 124B of the second specimen path 12B. The detector electrode 149 is provided for determining whether the second specimen path 12B is filled up with the specimen 30A, and also it is provided for determining whether the first specimen path 12A and second specimen path 12B have been filled up, thereby determining whether the test strip 10A operates normally. The determining method will be further described.

As shown in FIG.16B, in an embodiment of the present invention, the first electrode set 14A of the test strip 10A further comprises a third electrode 148A. When the specimen 30A flows through the third electrode 148A and the first reference electrode 146A, a third impulse signal is generated and is used together with the first impulse signal and the second impulse signal to obtain the flow time of the specimen 30A, threby obtaining the viscosity of the specimen 30A. As shown in FIG.16A, the third electrode 148A is disposed between the first electrode 142A and the second electrode 144A; however, the third electrode 148A can be disposed close to the first electrode 142A as well. Besides, in FIG.16B, the first electrode set 14A, the second electrode set 14B, the redox reagent 16A, the reaction reagent 16B, the first specimen path 12A, and the second specimen path 12B can be configured differently.

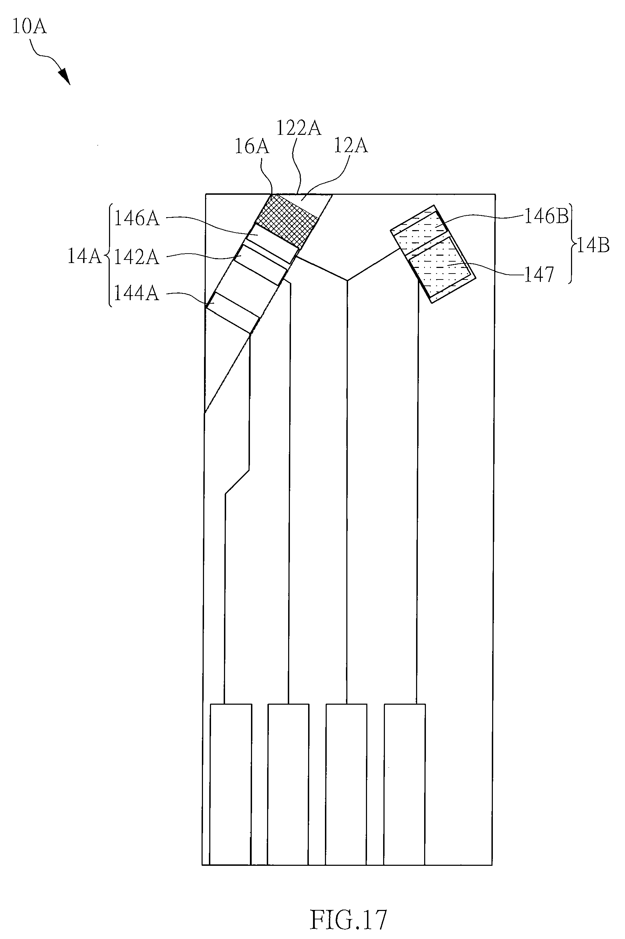

As shown in FIG. 17, in an embodiment of the present invention, the first reference electrode 146A of the first electrode set 14A of the test strip 10A and the second reference electrode 146B of the second electrode set 14B are the same electrode to save one reference electrode. As shown in FIG.17, in an embodiment of the present invention, the redox reagent 16A can be disposed near the inlet end 122A of the first specimen path 12A and in front of the first electrode set 14A. When the specimen 30A enters the first specimen path 12A, it first makes contact with the redox pair of the redox reagent 16A, then it carries and dissolves the redox pair in the redox reagent 16A and then makes contact with the the first electrode set 14A.

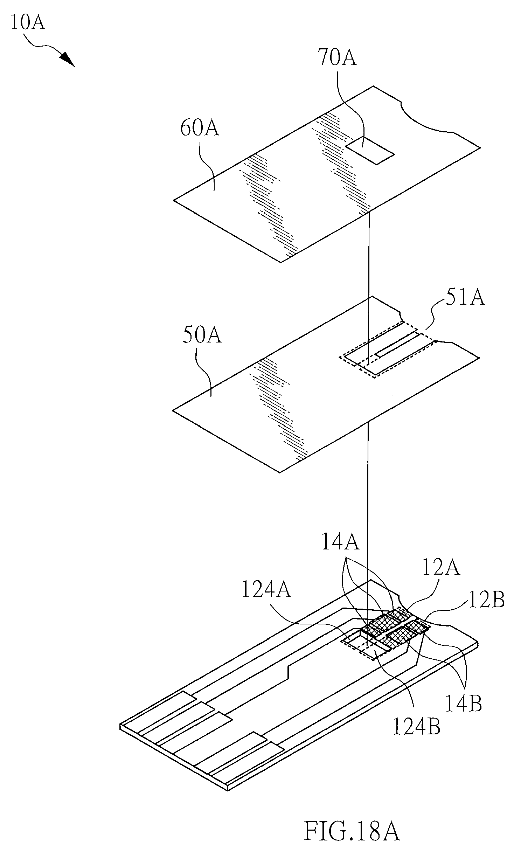

As shown in FIG.18A, in an embodiment of the present invention, the test strip 10A of the present invention comprises a substrate 40A, a spacer layer 50A and a cover layer 60A. In the embodiment, the first electrode set 14A and the second electrode set 14B are disposed on the substrate 40A; the spacer layer 50A covers the substrate 40A and exposes a portion of the first electrode set 14A and the second electrode set 14B; and the cover layer 60A covers the spacer layer 50A, thereby forming the first specimen path 12A and the second specimen path 12B.

As shown in FIG.18A, in an embodiment of the present invention, a breach 51A is form in the spacer layer 50A to correspond to the shape of the first specimen path 12A and the second specimen path 12B, thereby allowing the specimen 30 to flow in the first specimen path 12A and the second specimen path 12B. Furthermore, the test strip 10A comprises a through hole 70A penetrating through the substrate 40A, the spacer layer 50A, and the cover layer 60A to communicate with the discharge end 124A of the first specimen path 12A and the discharge end 124B of the second specimen path 12B, thereby increasing the area for discharging air and facilitating flowing of the specimen 30A. The through hole is disposed to stop the specimen 30A at the discharge end, so the specimen 30A will flow in the capillary and will not be drawn by the cover layer 60A or the substrate 40A to leave the capillary. It is noted that the present invention can have discharge holes disposed on the cover layer 60A or the substrate 40A respectively without using the through hole 70 and can still serve the purpose.

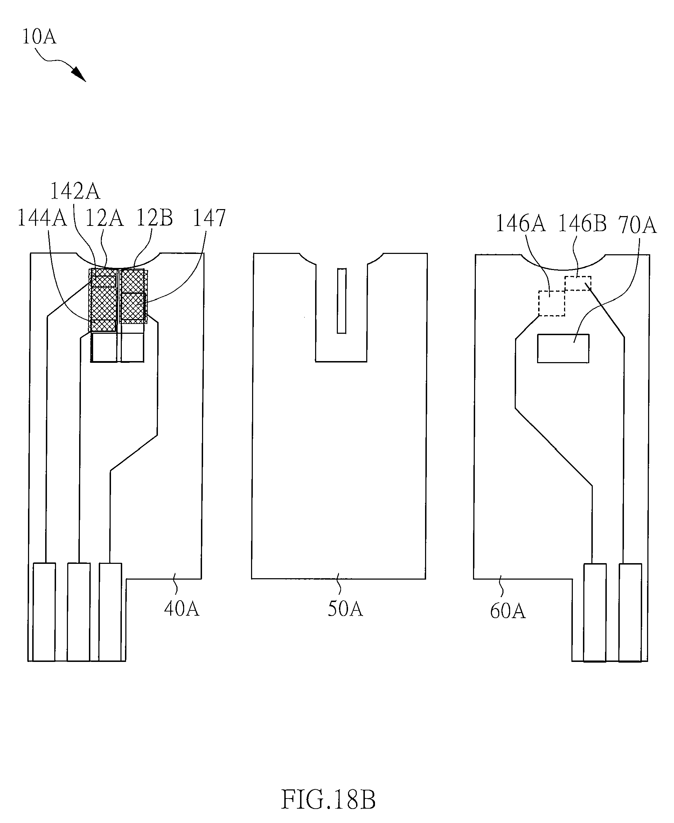

As shown in FIG.18B, in an embodiment of the present invention, the test strip 10A of the present invention comprises a substrate 40A, a spacer layer 50A and a cover layer 60A. In the embodiment, the first electrode 142A of the first electrode set 14A and the working electrode 147 of the second electrode set 14B are disposed on the substrate 40A; the spacer layer 50A covers the substrate 40A and exposes a portion of the first electrode 142A of the first electrode set 14A and the working electrode 147 of the second electrode set 14B; and the cover layer 60A covers the spacer layer 50A, thereby forming the first specimen path 12A and the second specimen path 12B. Besides, the first reference electrode 146A of the first electrode set 12A and the second reference electrode 146B of the second electrode set 12B are disposed on a lower surface of the cover layer 60A. The first reference electrode 146A and the second reference electrode 146B can be the same electrode as shown in FIG.17.

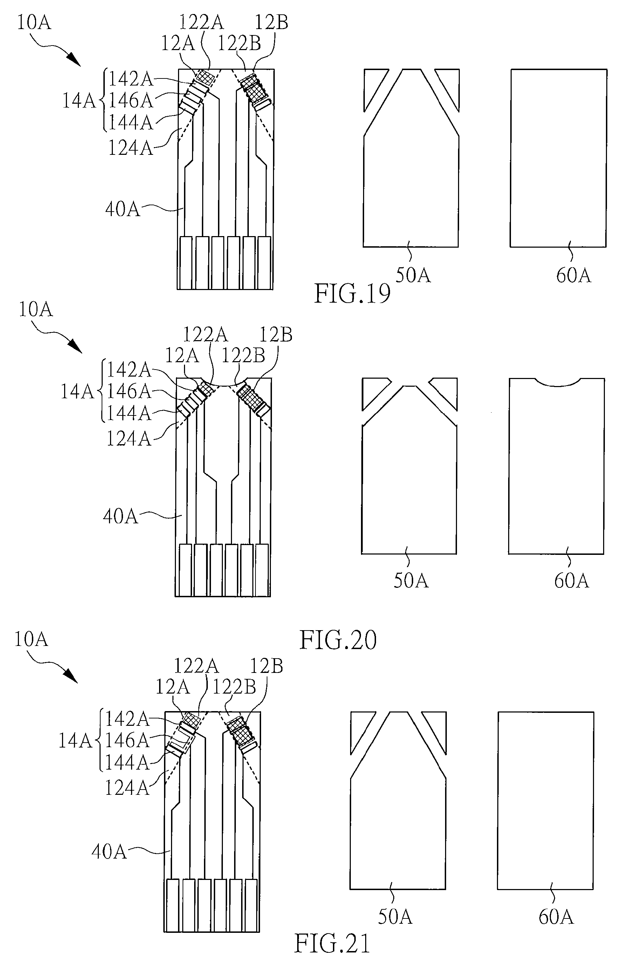

Furthermore, as shown in FIG.19 to FIG.26, in an embodiment of the present invention, the first specimen path 12A and the second specimen path 12B can be arranged in a V or Y shape; furthermore, the inlet end 122A of the first specimen path 12A does not communicate with the inlet end 122B of the second specimen path 12B, but the inlet end 122A of the first specimen path 12A can be disposed near the inlet end 122B to let the specimen 30A eneter the first specimen path 12A and the second specimen path 12B respectively at the same time.

As shown in FIG.19 to FIG.26, in an embodiment of the present invention, the first electrode 142A of the first specimen path 12A is disposed near the inlet end 122A of the first specimen path 12A, the second electrode 144A is disposed near the discharge end 124A of the first specimen path 12A, and the first reference electrode 146A is disposed between the first electrode 142A and the second electrode 144A (as shown in FIG.19, FIG.20, FIG.23 and FIG.24); or the first reference electrode 146A is formed in a fork shape having two ends disposed near the first electrode 142A and the second electrode 144A respectively (as shown in FIG.21, FIG.22, FIG.25, and FIG.26).

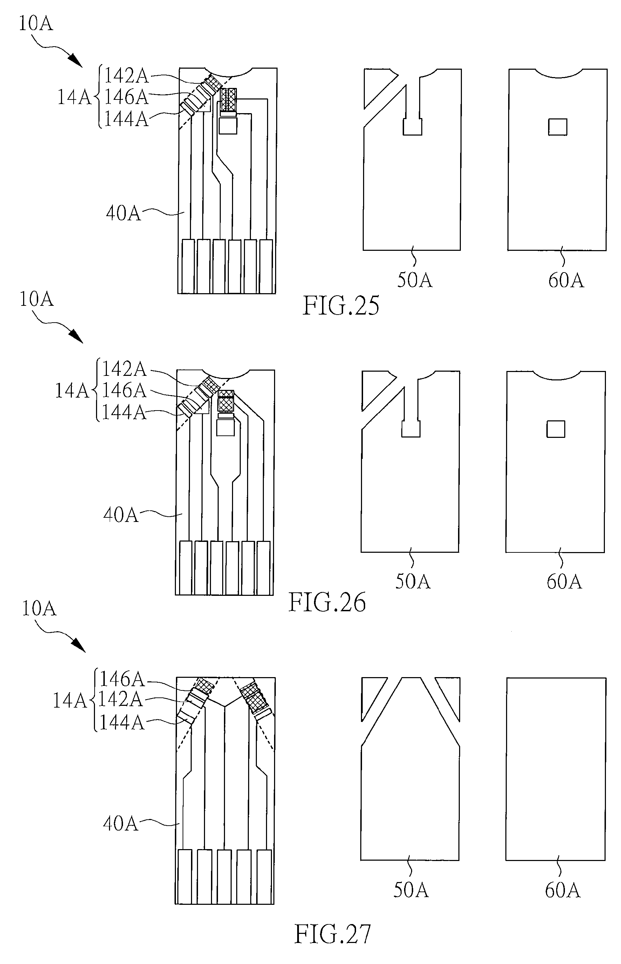

As shown in FIG.27 to FIG.30, in an embodiment of the present invention, the present invention can assign the first reference electrode 146A and the second reference electrode 146B to be the same electrode to save one electrode.

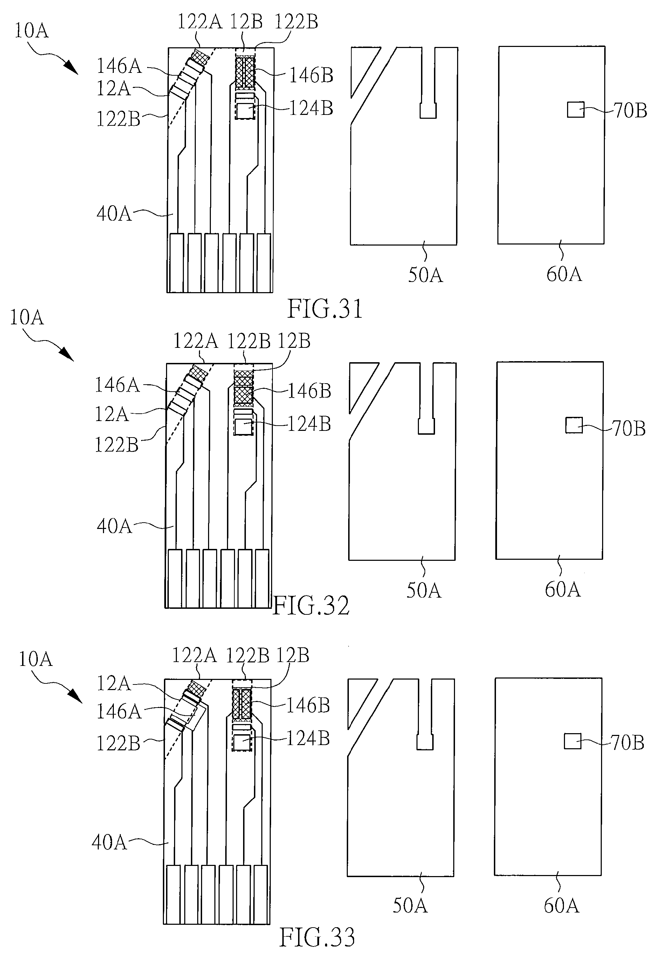

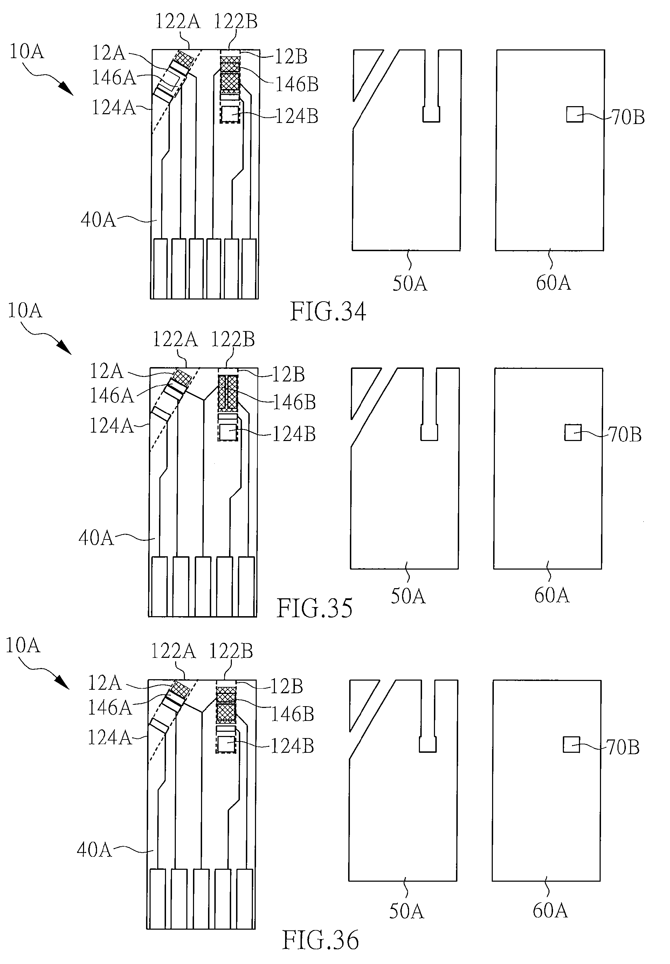

A shown in FIG.31 to FIG.36, in an embodiment of the present invention, the first specimen path 12A is extended inclinedly from the front end of the test strip 10A to the side of the test strip 10A; the second specimen path 12B can be extended perpendicularly from the front end of the test strip 10A towards the back end. The inlet end 122A of the first specimen path 12A does not communicate with the inlet end 122B of the second specimen path 12B; and the discharge end 124A does not communicate with the discharge end 124B as well; thereby forming two separate paths. Therefore, the specimen 30A can enter the first specimen path 12A and the second specimen path 12B respectively and the two paths do not interfere with each other. According to the structures shown in FIG.31 to FIG.36, since the first specimen path 12A is extended inclinedly from the front end of the test strip 10A to the side of the test strip 10A, the discharge end 124A of the first specimen path 12A can be directly connected to the open end on the side of the test strip 10A to discharge air. Therefore, only a through hole 70B is required to be formed on the cover layer 60A to communicate with the spacer layer 50A and the discharge end 124B of the second specimen path 12B on the substrate 40A to discharge air. The first reference electrode 146A and the second reference electrode 146B can be disposed separartely (as shown in FIG.31 to FIG.34); or the first reference electrode 146A and the second reference electrode 146B can be the same electrode (as shown in FIG.35 and FIG36).

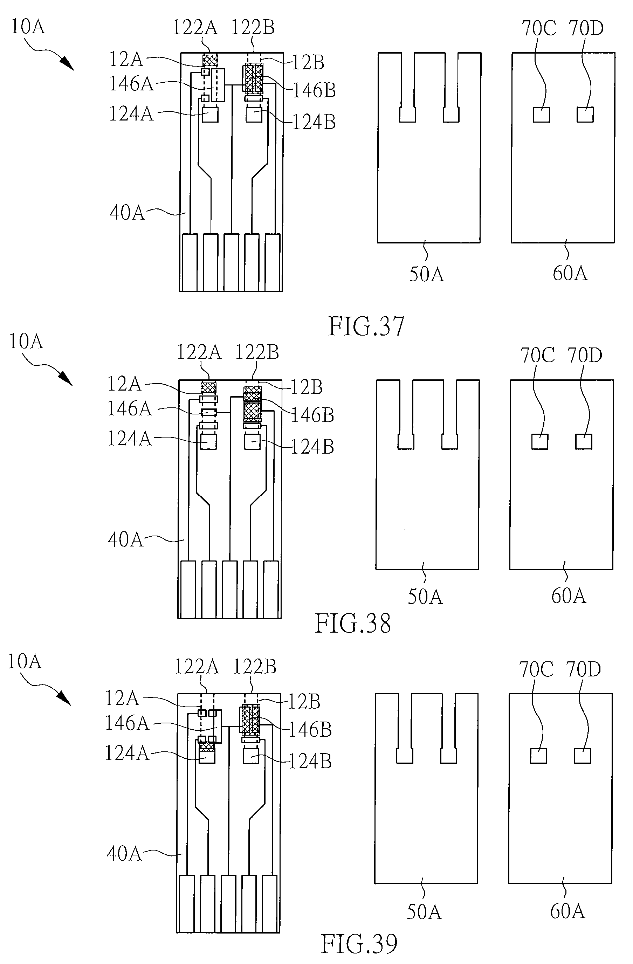

As shown in FIG.37 to FIG.41, in an embodiment of the present invention, the first specimen path 12A can be extended perpendicularly from the front end of the test strip 10A towards the back end, and the second specimen path 12B can also be extended perpendicularly from the front end of the test strip 10A towards the back end. The inlet end 122A of the first specimen path 12A does not communicate with the inlet end 122B of the second specimen path 12B; and the discharge end 124A does not communicate with the discharge end 124B as well; thereby allowing the specimen 30A to enter the first specimen path 12A and the second specimen path 12B respectively. According to the structures shown in FIG.37 to FIG.41, two through holes 70C and 70D are required to be formed on the cover layer 60A to communicated with the spacer layer 50A, the discharge end 124A of the first specimen path 12A of the substrate 40A, and the discharge end 124B of the second specimen path 12B of the substrate 40A to discharge air. The first reference electrode 146A and the second reference electrode 146B can be the same electrode.

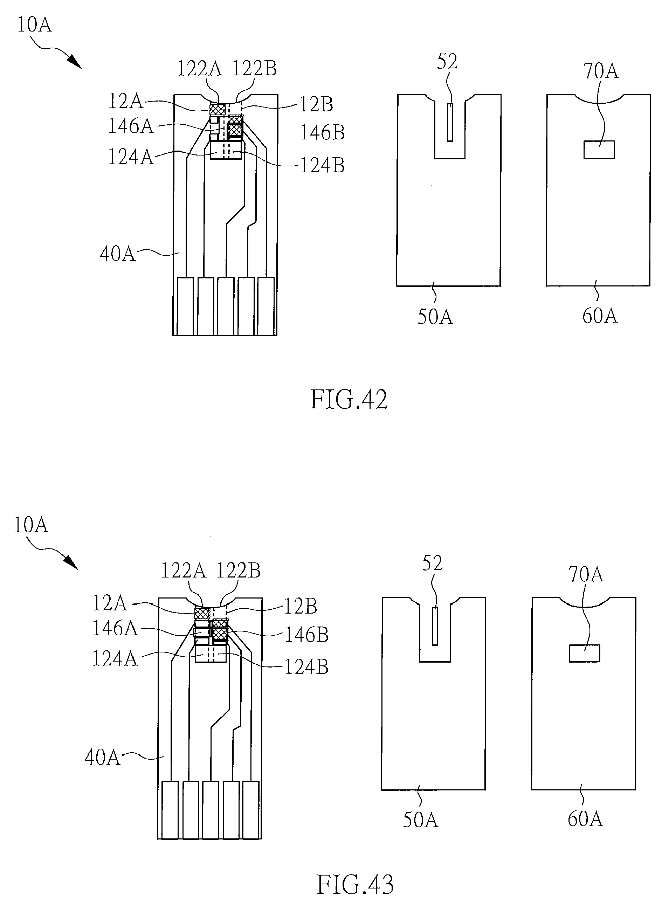

As shown in FIG.42 to FIG.48, in an embodiment of the present invention, the first specimen path 12A can be extended perpendicularly from the front end of the test strip 10A towards the back end, and the second specimen path 12B can also be extended perpendicularly from the front end of the test strip 10A towards the back end. The inlet end 122A of the first specimen path 12A is disposed near the inlet end 122B of the second specimen path 12B, so the specimen 30A are drawn by the inlet end 122A and inlet end 122B at the same time. The discharge end 124A also communicates with the discharge end 124B. A spacing bar 80 is disposed in the spacer layer 50A to separate the first specimen path 12A and the second specimen path 12B so as to let the specimen 30A enter the first specimen path 12A and the second specimen path 12B respectively. According to the structures shown in FIG.42 to FIG.48, only a through hole 70A is required to be formed on the cover layer 60A to communicate with the discharge end 124A of the first specimen path 12A on the substrate 40A and the discharge end 124B of the second specimen path 12B on the substrate 40A to discharge air. The first reference electrode 146A and the second reference electrode 146B can be the same electrode.

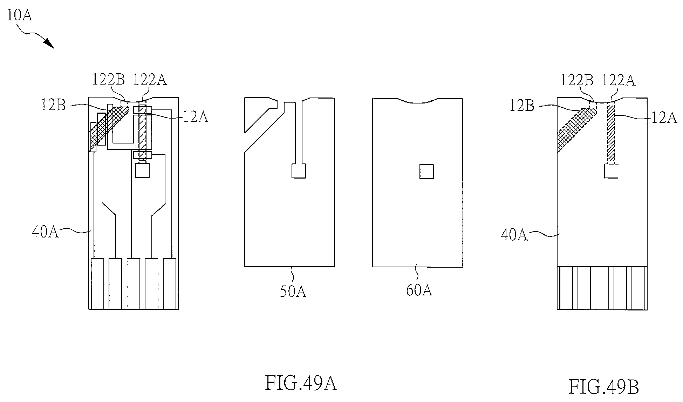

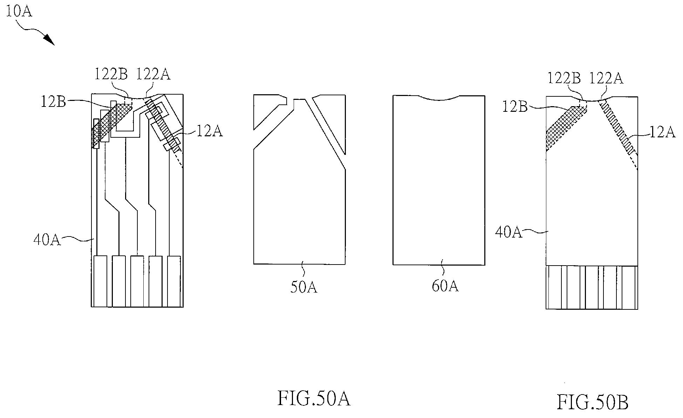

Please refer to FIG.49A to FIG.50B for a preferred embodiment of the test strip 10A of the present invention. FIG.49A and FIG.50A illustrate the substrate 40A, the spacer layer 50A, and the cover layer 60A of the test strip 10A; while the FIG.49B and FIG.50B illlustrate the combinations of substrate 40A, the spacer layer 50A and the cover layer 60A of the FIG.49A and FIG.50A respectively. From the experiment, it can be seen that if the first specimen path 12A and the second specimen path 12B need to receive the specimen 30A at the same time, then the inlet ends 122A and 122B should have the same or similar width, and the two inlet ends 122A, 122B should be apart from each other, wherein the width of each inlet end is not closely related to the whole width of the specimen path. The preferred width of the two inlet ends is about 0.2 to 1 mm, and the preferred spacer of the two inlet ends is about 0.01 to 1.5 mm. If the two inlet ends of the specimen paths have different width, then it is easier for the specimen 30A to pass through the larger inlet end and it is not easy for the specimen 30A to pass through the smaller inlet end; therefore, the specimen 30A would not enter the inlet ends at the same time. Preferably, the two inlet ends of the specimen paths are not connected, instead, they are separated to let the specimen 30A enter both inlet ends. Therefore, as shown in FIG.49A to FIG.50B, in an preferred embodiment of the present invention, the inlet end 122A of the first specimen path 12A and the inlet end 122B of the second specimen path 12B have substantially the same width for the specimen 30A to enter at the same time. As shown in FIG.49A and FIG.49B, the first specimen path 12A can be extended from the front end of the test strip 10A towards the opposing end; alternatively, as shown in the FIG.50A and FIG.50B, the first specimen path 12A can be extended inclinedly from the front end of the test strip 10A to a side of the test strip 10A.

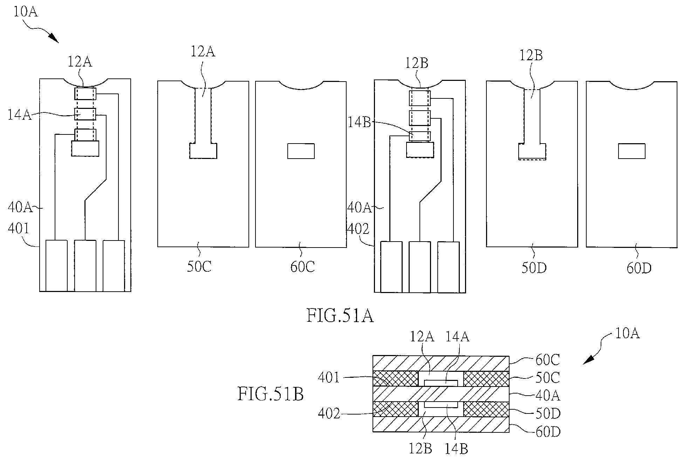

As shown in FIG.51A to FIG.59B, the first electrode set 14A and the second electrode set 14B can be formed in a stack configuration, wherein the first electrode set 14A and the second electrode set 14B are disposed on different planes; also, the first specimen path 12A and the second specimen path 12B can be formed in a stack configuration, wherein the first specimen path 12A and the second specimen path 12B are disposed on different planes. FIG.51A, FIG.52A, FIG.53A, FIG.54A, FIG.55A, FIG.56A, FIG.57A, FIG.58A, and FIG.59A illustrate the test strip according to an embodiment of the present invention in a stack configuration; while FIG.51B, FIG.52B, FIG.53B, FIG.54B, FIG.55B, FIG.56B, FIG.57B, FIG.58B, and FIG.59B illustrate the sectional views of the FIG.51A, FIG.52A, FIG.53A, FIG.54A, FIG.55A, FIG.56A, FIG.57A, FIG.58A, and FIG.59A respectively.

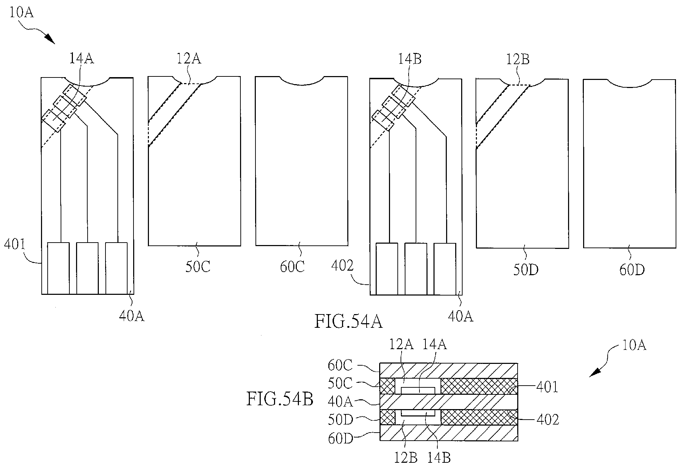

As shown in FIG.51A, FIG.54A, FIG.51B, and FIG.54B, in an embodiment of the present invention, the test strip 10A comprises the substrate 40A, the first spacer layer 50C, the first cover layer 60C, the second spacer layer 50D, and the second cover layer 60D. The substrate comprises a first surface 401 and a second surface 402, wherein the first electrode set 14A is disposed on the first surface 401, and the second electrode set 14B is disposed on the second surface 402; the first spacer layer 50C covers the first surface 401 of the substrate 40A and exposes a portion of the first electrode set 14A; the first cover layer 60C covers the first spacer layer 50C to form the first specimen path 12A; the second spacer layer 50D covers the second surface 402 of the substrate 40A and exposes a portion of the second electrode set 14B; and the second cover layer 60D covers the second spacer layer 50D to form the second specimen path 12B. Therefore, the first specimen path 12A and the second specimen path 12B are formed in a vertical stack configuration. In an embodiment of the present invention, the first specimen path 12A and the second specimen path 12B can be extended from the front end of the test strip 10A towards the opposing end (as shown in FIG.51A and FIG.51B); or the first specimen path 12A and the second specimen path 12B can be extended inclinedly from a front end of the test strip 10A towards a side (as shown in FIG.54A and FIG.54B).

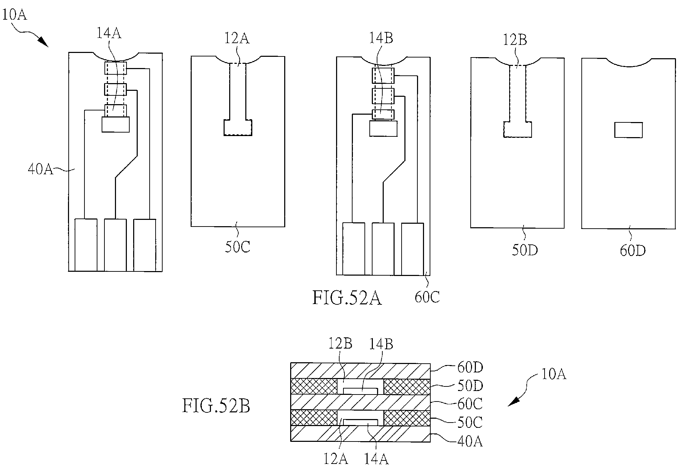

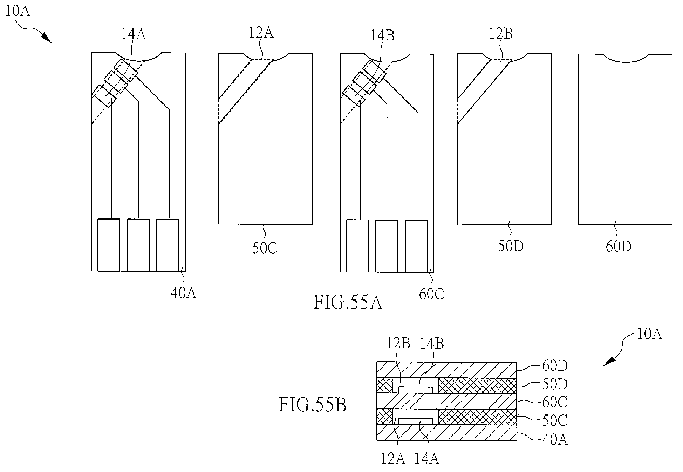

As shown in FIG.52A, FIG.55A, FIG.52B, and FIG.55B, in an embodiment of the present invention, the test strip 10A comprises the substrate 40A, the first spacer layer 50C, the first cover layer 60C, the second spacer layer 50D, and the second cover layer 60D. The first electrode set 14A is disposed on the substrate 40A; the first spacer layer 50C covers the substrate 40A and exposes a portion of the first electrode set 14A; the first cover layer 60C covers the first spacer layer 50C to form the first specimen path 12A; the second electrode set 14B is disposed on the first cover layer 60C; the second spacer layer 50D covers the first cover layer 60C and exposes a portion of the second electrode set 14B; and the second cover layer 60D covers the second spacer layer 50D to form the second specimen path 12B. Therefore, the first specimen path 12A and the second specimen path 12B are formed in a vertical stack configuration. In an embodiment of the present invention, the first specimen path 12A and the second specimen path 12B can be extended from the front end of the test strip 10A towards the opposing end (as shown in FIG.52A and FIG.52B); or the first specimen path 12A and the second specimen path 12B can be extended inclinedly from a front end of the test strip 10A towards a side (as shown in FIG.55A and FIG.55B).

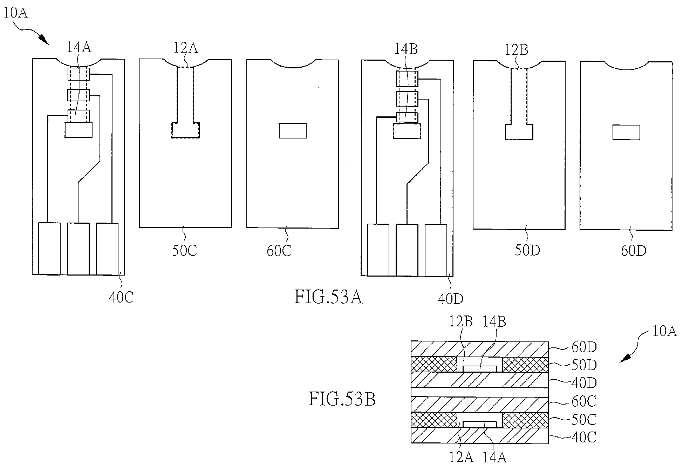

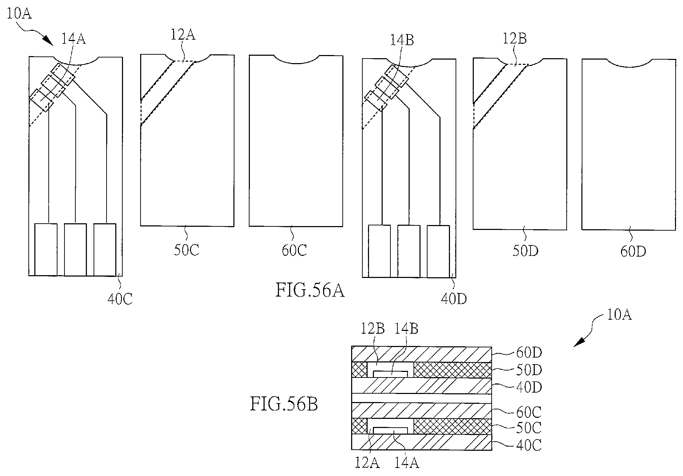

As shown in FIG.53A, FIG.56A, FIG.53B, and FIG.56B, in an embodiment of the present invention, the test strip 10A comprises the first substrate 40C, the first spacer layer 50C, the first cover layer 60C, the second spacer layer 50D, and the second cover layer 60D. The first electrode set 14A is disposed on the first substrate 40C; the first spacer layer 50C covers the first substrate 40C and exposes a portion of the first electrode set 14A; the first cover layer 60C covers the first spacer layer 50C to form the first specimen path 12A; the second electrode set 14B is disposed on the second substrate 40D; the second spacer layer 50D covers the second substrate 40D and exposes a portion of the second electrode set 14B; and the second cover layer 60D covers the second spacer layer 50D to form the second specimen path 12B. Then an adhesive layer is used to attach the first cover layer 60C and the second substrate 40D. Therefore, the first specimen path 12A and the second specimen path 12B are formed in a vertical stack configuration. In an embodiment of the present invention, the first specimen path 12A and the second specimen path 12B can be extended from the front end of the test strip 10A towards the opposing end (as shown in FIG.53A and FIG.53B); or the first specimen path 12A and the second specimen path 12B can be extended inclinedly from a front end of the test strip 10A towards a side (as shown in FIG.56A and FIG.56B).

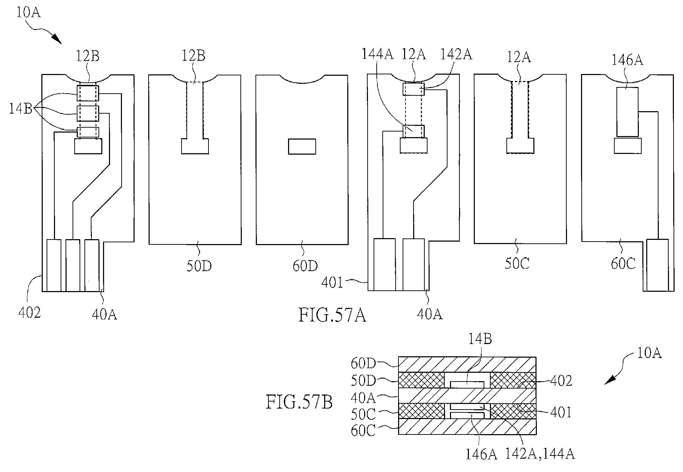

As shown in FIG.57A and FIG.57B, in an embodiment of the present invention, the test strip 10A comprises the substrate 40A, the first spacer layer 50C, the first cover layer 60C, the second spacer layer 50D, and the second cover layer 60D. The substrate comprises a first surface 401 and a second surface 402, wherein the first electrode 142A of the first electrode set 14A is disposed on the first surface 401, and the second electrode set 14B is disposed on the second surface 402; the first spacer layer 50C covers the first surface 401 of the substrate 40A and exposes a portion of the first electrode 142A and the second electrode 144A of first electrode set 14A; the first cover layer 60C covers the first spacer layer 50C, and the first reference electrode 146A of the first electrode set 14 is disposed on the upper surface of the first cover layer 60C, thereby forming the first specimen path 12A; the second spacer layer 50D covers the second surface 402 of the substrate 40A and exposes a portion of the second electrode set 14B; and the second cover layer 60D covers the second spacer layer 50D to form the second specimen path 12B. Therefore, the first specimen path 12A and the second specimen path 12B are formed in a vertical stack configuration.

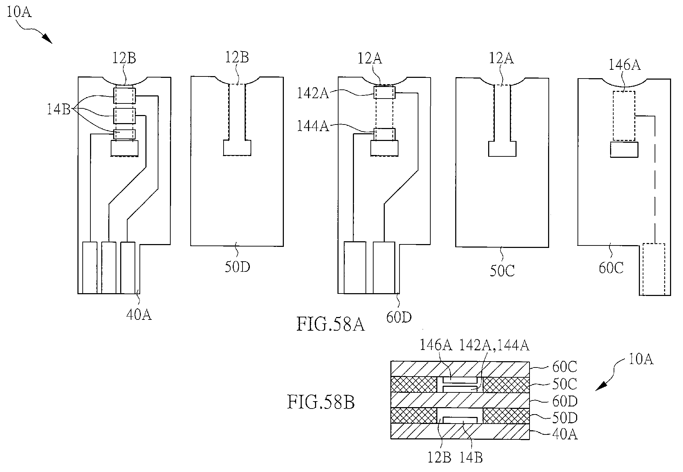

As shown in FIG.58A and FIG.58B, in an embodiment of the present invention, the test strip 10A comprises the substrate 40A, the first spacer layer 50C, the first cover layer 60C, the second spacer layer 50D, and the second cover layer 60D. The second electrode set 14B is disposed on the substrate 40A; the second spacer layer 50D covers the substrate 40A and exposes a portion of the second electrode set 14B; the second cover layer 60D covers the second spacer layer 50D to form the second specimen path 12B; the first electrode 142A and the second electrode 144A of the first electrode set 14A are disposed on the second cover layer 60D; the first spacer layer 50C covers the second cover layer 60D and exposes a portion of the first electrode 142A and the second electrode 144A of the first electrode set 14A; the first cover layer 60C covers the first spacer layer 50C and has the first reference electrode 146A of the first electrode set 14A disposed on a lower surface of the first cover layer 60C to form the first specimen path 12A. Therefore, the first specimen path 12A and the second specimen path 12B are formed in a vertical stack configuration.

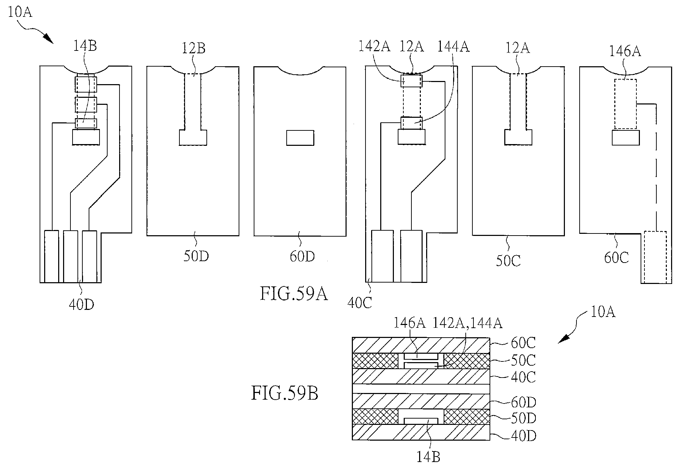

As shown in FIG.59A and FIG.59B, in an embodiment of the present invention, the test strip 10A comprises the first substrate 40C, the first spacer layer 50C, the first cover layer 60C, the second substrate 40D, the second spacer layer 50D, and the second cover layer 60D. The second electrode set 14B is disposed on the second substrate 40D; the second spacer layer 50D covers the second substrate 40D and exposes a portion of the second electrode set 14B; the second cover layer 60D covers the second spacer layer 50D to form the second specimen path 12B; the first electrode 142A and the second electrode 144A of the first electrode set 14A are disposed on the first substrate 40C; the first spacer layer 50C covers the first substrate 40C and exposes a portion of the first electrode 142A and the second electrode 144A of the first electrode set 14A; the first cover layer 60C covers the first spacer layer 50C and has the first reference electrode 146A of the first electrode set 14A disposed on a lower surface of the first cover layer 60C to form the first specimen path 12A. Then an adhesive layer is used to attach the second cover layer 60D and the first substrate 40C. Therefore, the first specimen path 12A and the second specimen path 12B are formed in a vertical stack configuration.

Furthermore, in the present invention, the first specimen path 12A and the second specimen path 12B can have different widths. In an embodiment of the present invention, in order to let the specimen 30A enter the inlet ends at the same time, the inlet end 122A of the first specimen path 12A and the inlet end 12B of the second specimen path 12B are separated and have the same width, and the inlet end 122A of the first specimen path 12A should be 0.01 to 1.5 mm apart from the inlet end 122B of the second specimen path 12B. When the specimen 30A is blood, the volume of the first specimen path 12A is approximately 0.1 to 1 micro liter, the length of the first specimen path 12A is approximately 5 to 15 mm, and the width of the first specimen path 12A is approximately 0.2 to 2 mm.

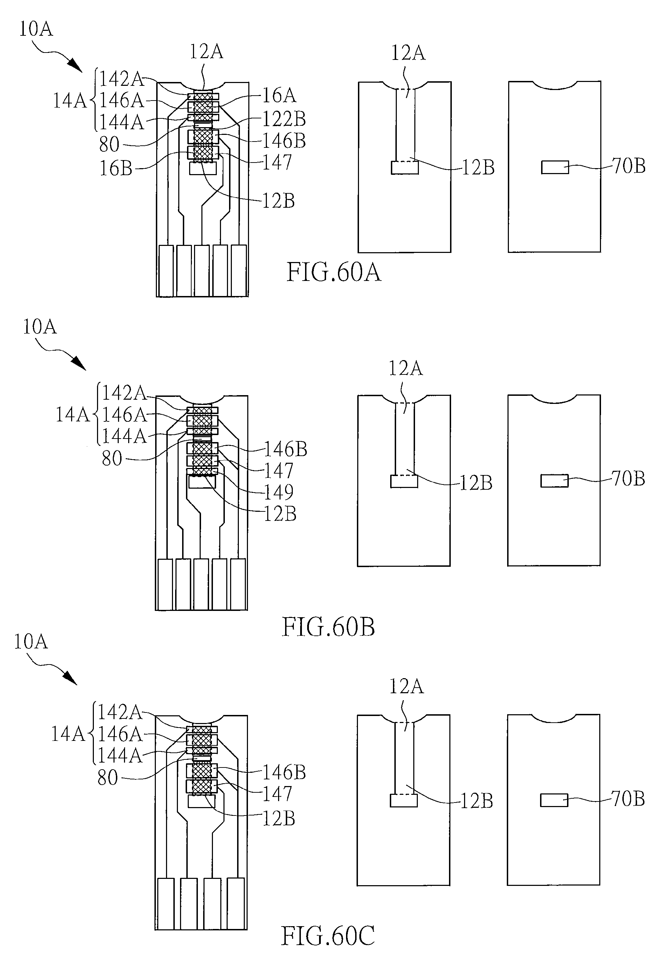

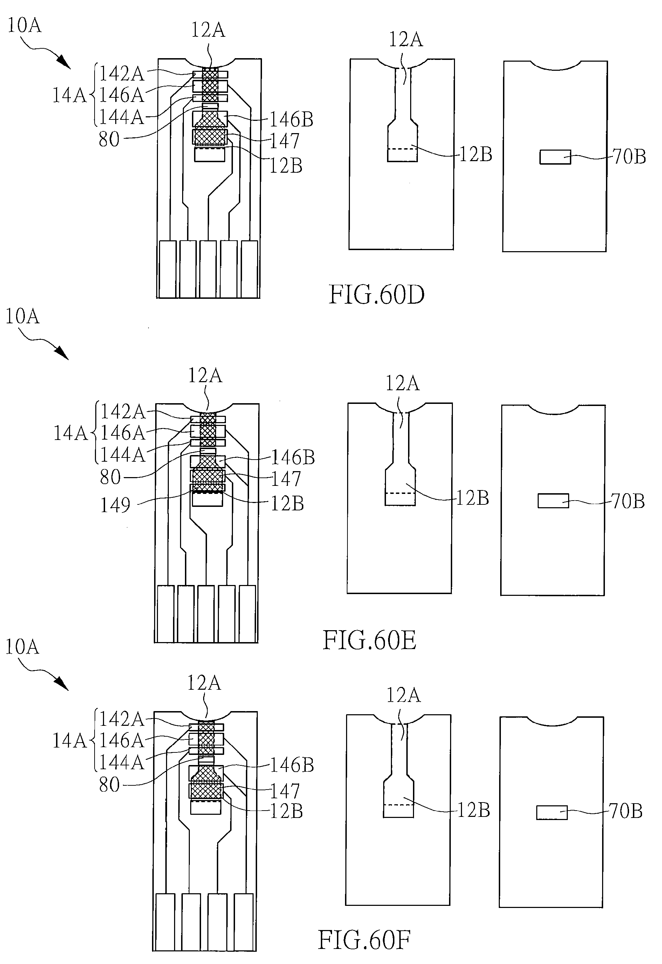

Additionally, as shown in FIG.60A to FIG.62H, in an embodiment of the present invention, the test strip 10A of the present invention can have the inlet end 122B of the second specimen path 12B connected to the discharge end 124A of the first specimen path 12A to let the first specimen path 12A and the second specimen path 12B form a series mode, wherein the first specimen path 12A and the second specimen path 12B have a insulating bar (or insulating layer) or a spacing bar 80 printed therebetween to keep the redox reagent 16A in the first specimen path 12A from mixing with the reaction reagent 16B in the second specimen path 12B. When the first specimen path 12A is in series with the second specimen path 12B, the flow time can be detected as the specimen 30A flows through the first specimen path 12A. When the specimen 30A is flowing in the first specimen path 12A, it does not make contact with the reaction reagent 16B disposed in the second specimen path 12B or only just a little of the reaction reagent 16B; therefore, the fluidity of the specimen 30A is not affected. On the other hand, since the specimen 30A first flows through the redox reagent 16A and then the reaction reagent 16B, the redox reagent 16A would increase the background signal of the concentration of the analyte, so the electrochemical instrument 20 is used to calculate the effect of the redox reagent 16A and eliminates it. In an embodiment of the present invention, only a tiny amount of redox reagent 16A is needed to keep it from affecting the concentration of the analyte. When the concentration of the redox reagent 16A is too low for the electrode to detect, then the voltage on the electrode can be increased to help detect the specimen 30A.

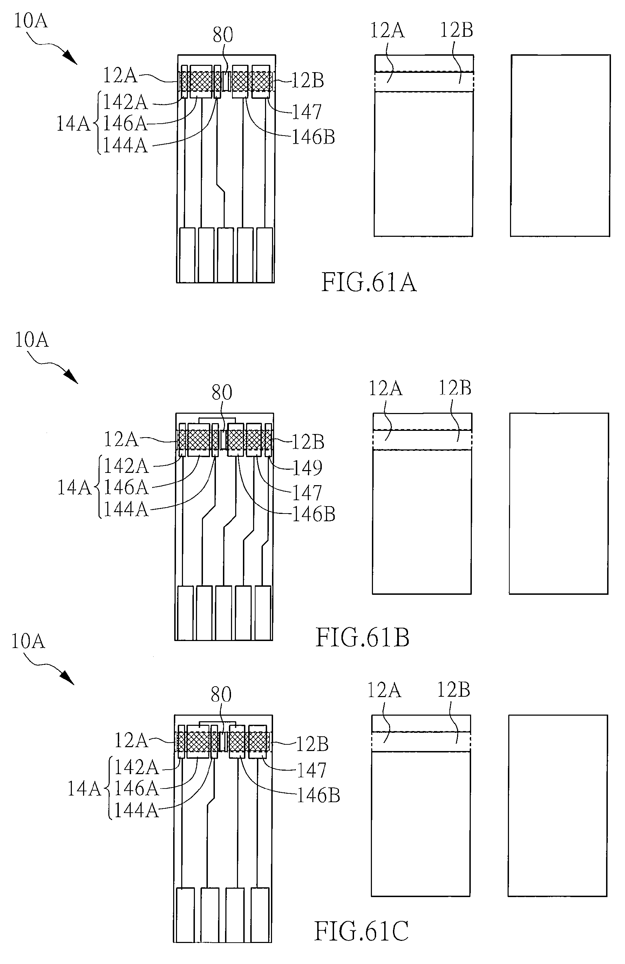

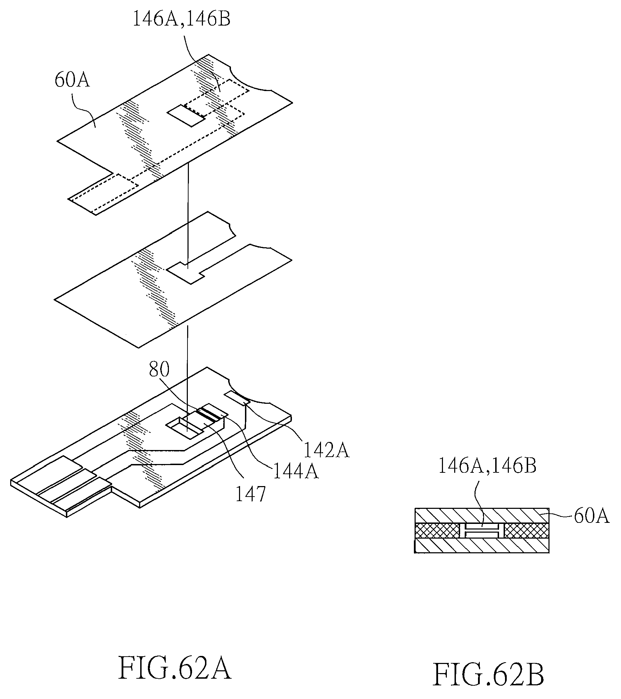

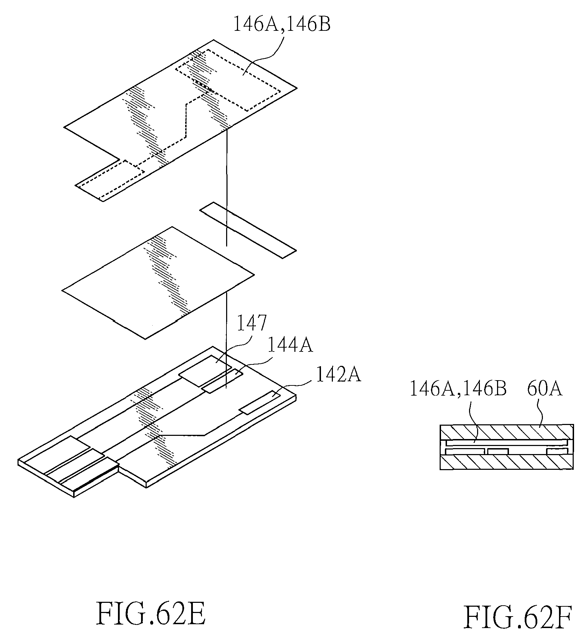

In an embodiment of the present invention, when the first specimen path 12A is in series with the second specimen path 12B, the inlet end 122A of the first specimen path 12A can be disposed at a front end (as shown in FIG.60A to FIG.60F and FIG.62A to FIG.62D) or a side (as shown in FIG.61A to FIG.61C and FIG.62E to FIG.62H) of the test strip 10A.

In an embodiment of the present invention, when first specimen path 12A is in series with the second specimen path 12B, the test strip 10A can also comprise a through hole 70B communicating with the discharge end 124B of the second specimen path 12B (as shown in FIG.60A to FIG.60F, and FIG.62A to FIG.62D); the first specimen path 12A and the second specimen path 12B can have the same width (as shown in FIG.60A to FIG.60C, FIG.61A to FIG.61C, and FIG.62A to FIG.62H); or the first specimen path 12A has a width smaller than that of the second specimen path 12B (as shown in FIG.60D to FIG.60F). Since the first specimen path 12A is provided for flow time detection, having a smaller width is easier to distinguish the different viscosity ranges; on the other hand, the second specimen path 12B is provided for detecting the concentration of the analyte, the response signal is proportional to the amount of specimen, having a larger width is easier to obtain more amount of specimen.

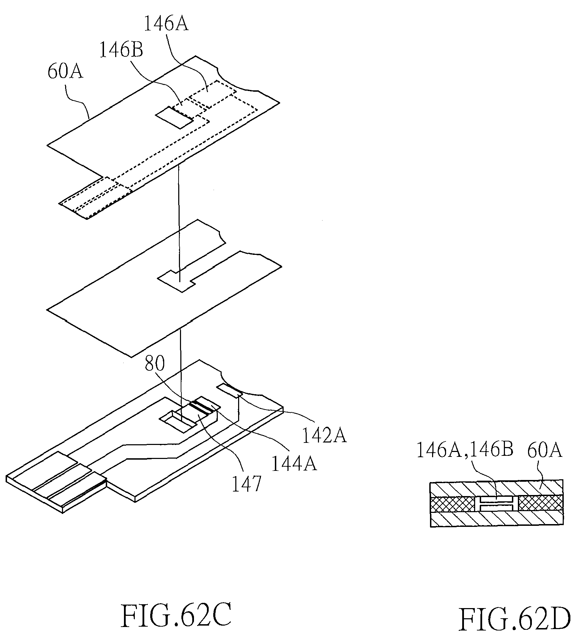

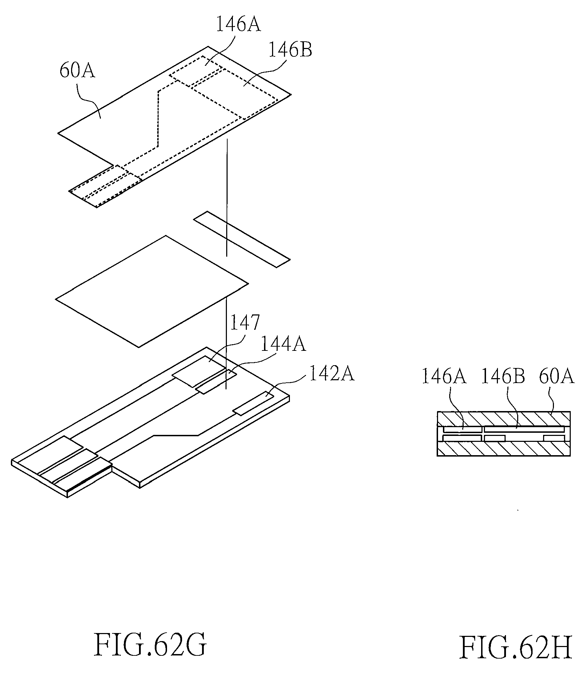

In an embodiment of the present invention, when the first specimen path 12A is in series with the second specimen path 12B, the first electrode set 14A of the test strip 10A can comprise the first electrode 142A, the second electrode 144A, and the first reference electrode 146A, and the second electrode set 14B can comprise the working electrode 147 and the second reference electrode 146B (as shown in FIG.60A, FIG.60C, FIG.60D, FIG.60F, FIG.61A, FIG.61C, FIG.62A to FIG.62H). The first reference electrode 146A and the second reference electrode 146B can be the same electrode (as shown in FIG.60C, FIG.60F, FIG.61C, FIG.62A, FIG.62B, FIG.62E, and FIG.62F). In an embodiment of the present invention, the first electrode set 14A can comprises the first electrode 142A, the second electrode 144A, and the first reference electrode 146A; the second electrode set 14B can comprise the working electrode 147, the second reference electrode 146B, and the detector electrode 149, wherein the first reference electrode 146A and the second reference electrode 146B can be the same electrode (as shown in FIG.60B, FIG.60E, and FIG.61B). Besides, in an embodiment of the present invention, the first reference electrode 146A and the second reference electrode 146B can be disposed on the lower surface of the cover layer 60A (as shown in FIG.62A to FIG.62H), wherein the first reference electrode 146A and the second reference electrode 146B can be the same electrode (as shown in FIG.62A, FIG.62B, FIG.62E, and FIG.62F), or they can be different electrode (as shown in FIG.62C, FIG.62D, FIG.62G, and FIG.62H).

In an embodiment of the present invention shown in FIG.60A to FIG.62H, since the first specimen path 12A comprises two detector electrodes (that is the first electrode 142A and the second electrode 144A), which can calculate the flow time respectively. Therefore, the flow time dectection can be done when the specimen 30A has flowed through the first specimen path 12A and hasn't made contact with the enzymes of the second specimen path 12B. Therefore, the flow time detection will not be affected by the enzymes and thus provides an accurate result.

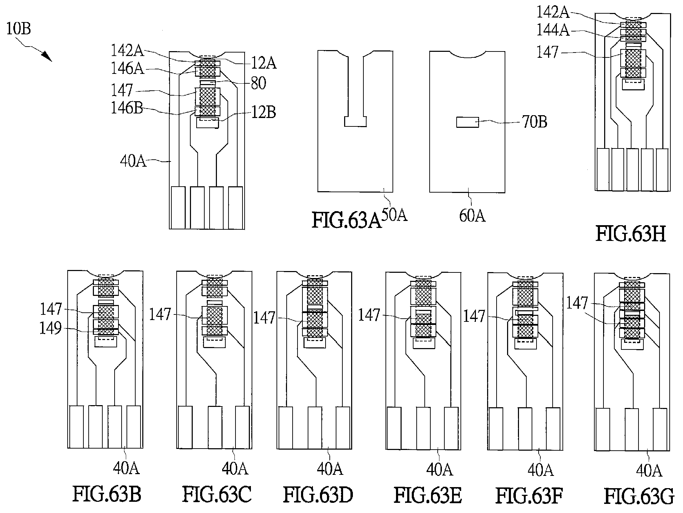

Additionally, as shown in FIG.63A to FIG.74B, the present invention provides a detecting device comprising a test strip 10B. The test strip 10B is similar to the test strip 10A shown in FIG.60A to FIG.62H in that the first specimen path 12A is also in series with the second specimen path 12B; however, the first electrode set 14A of the test strip 10B comprises the first electrode 142A and the first reference electrode 146A, the second electrode set 14B comprises the working electrode 147 and the second reference electrode 146B. When the specimen 30A is in contact with the first electrode 142A and the first reference electrode 146A, a first impulse signal is generated; when the specimen 30A is in contact with the first reference electrode 146A and the working electrode 147, a second impulse signal is generated; therefore, a flow time of the specimen 30A is obtained according to the first impulse signal and the second impulse signal. Compared with the test strip 10A shown in FIG.60A to FIG.62H, the test strip 10B comprises only one time detecting electrode (i.e, the first electrode 142A) and treats the working electrode 147 as the second time detecting electrode; therefore, the present invention can use one less electrode and still can have the first impulse signal and the second impulse signal generated. Besides, when using the test strip 10B to detect the flow time, the specimen 30A would be in contact with only a little amount of enzymes, thereby reducing the effect of the enzymes.

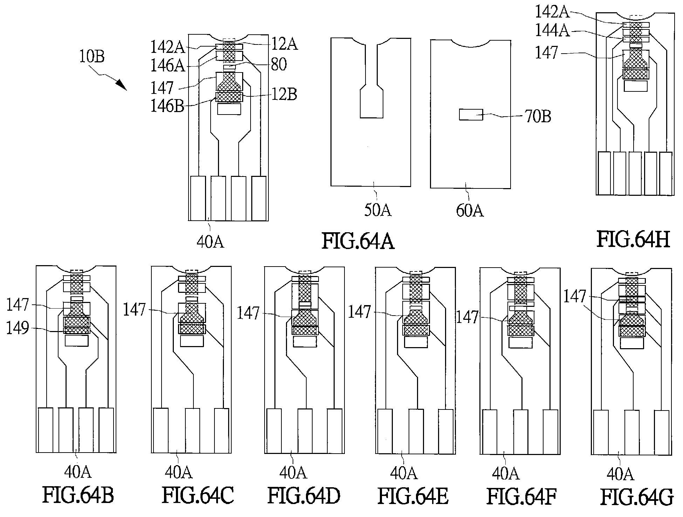

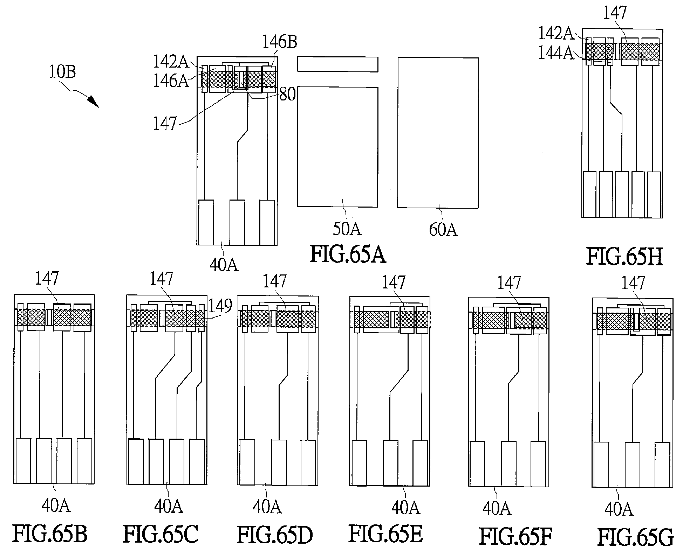

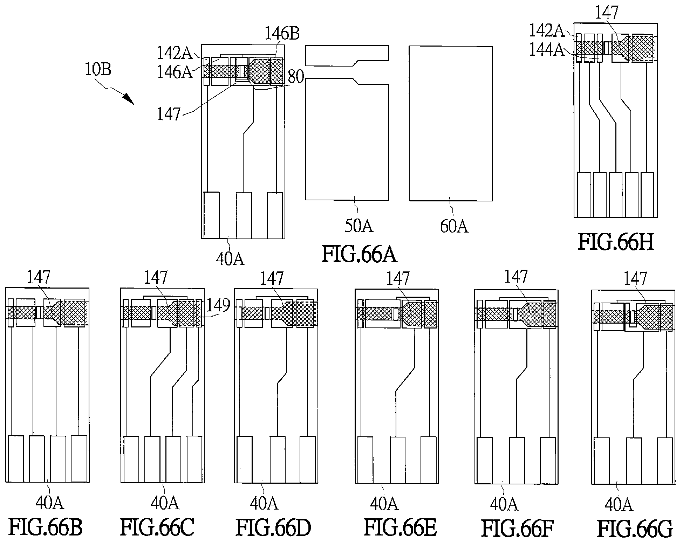

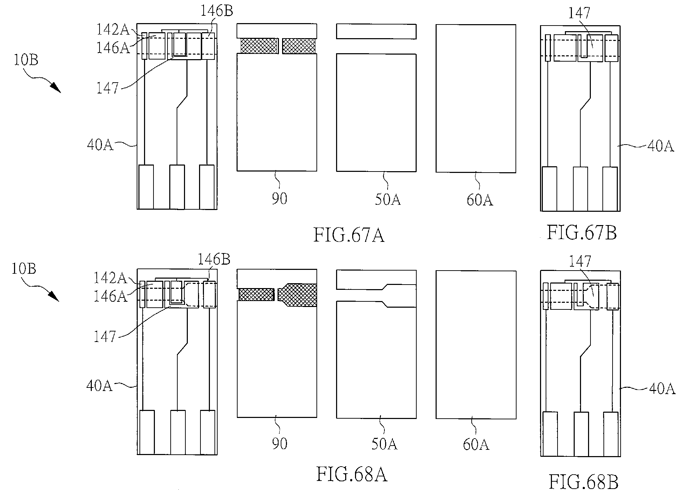

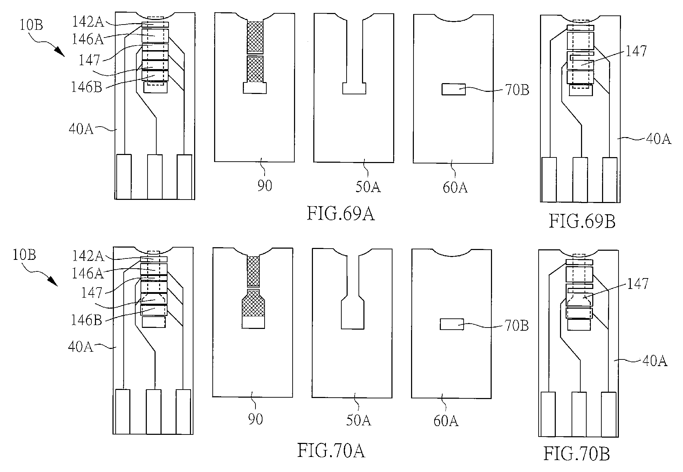

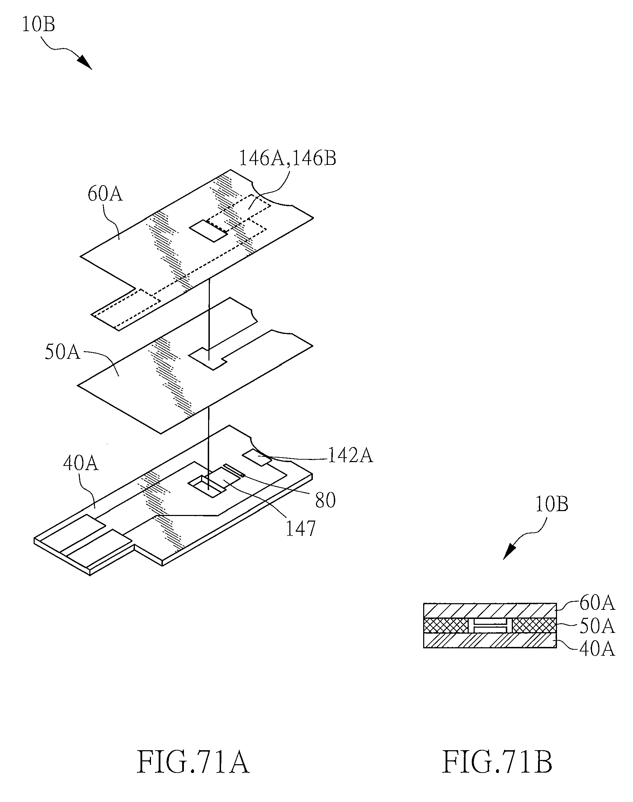

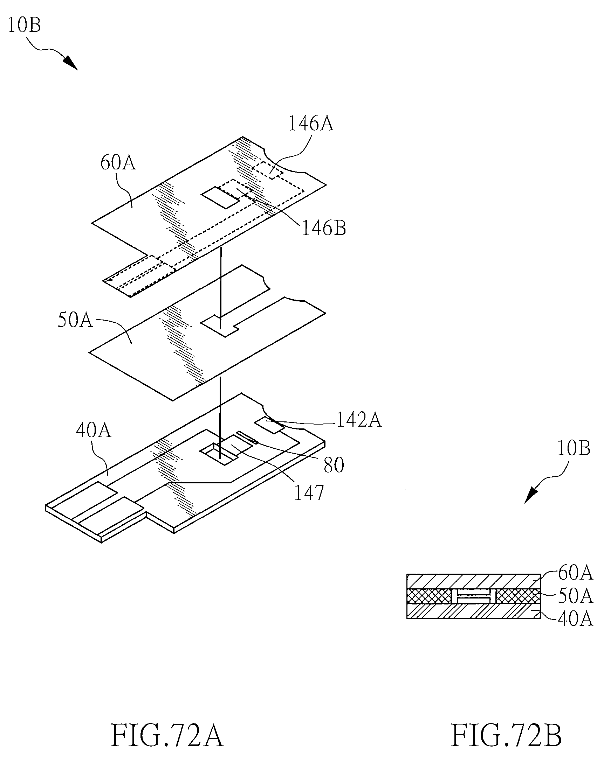

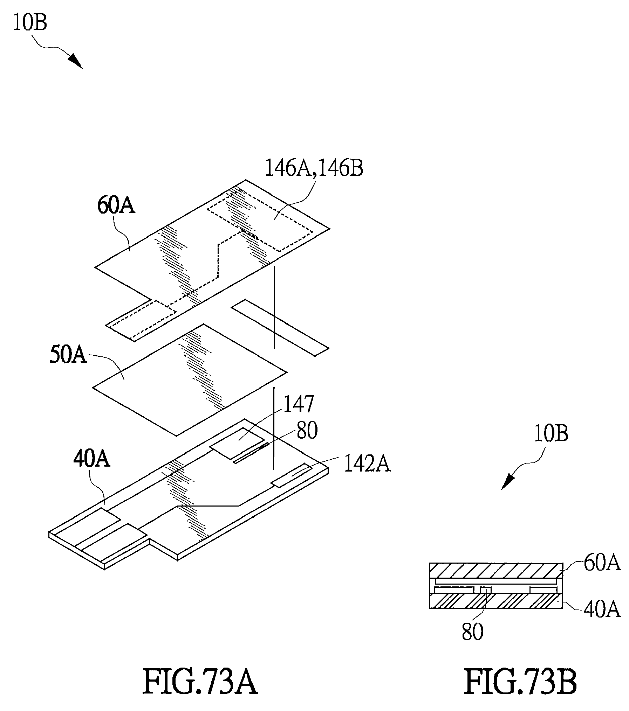

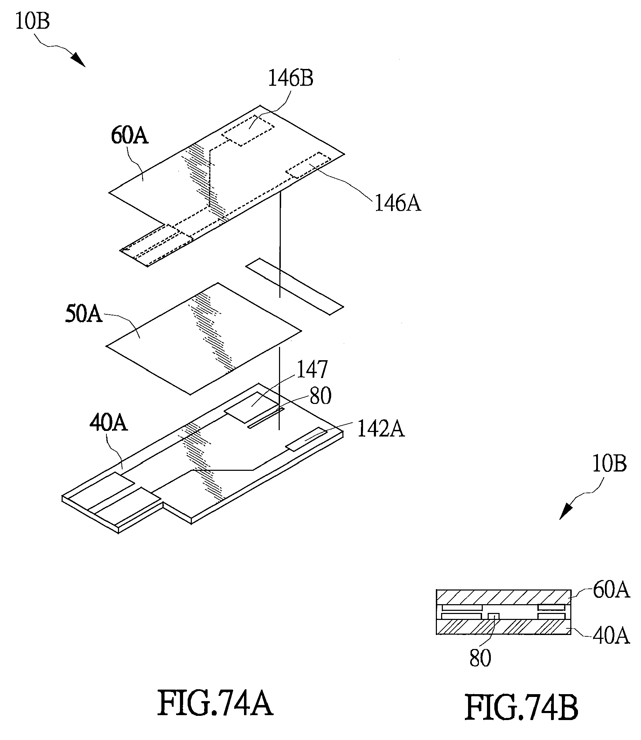

As shown in FIG.63A, FIG.64A, FIG.65A, and FIG.66A, the test strip 10B comprises the substrate 40A, the spacer layer 50A, and the cover layer 60A; FIG.63B to FIG.63H illustrate various embodiments of the substrate 40A shown in FIG.63A; FIG.64B to FIG.64H illustrate various embodiments of the substrate 40A shown in FIG.64A; FIG.65B to FIG.65H illustrate various embodiments of the substrate 40A shown in FIG.65A; FIG.66B to FIG.66H illustrate various embodiments of the substrate 40A shown in FIG.66A. As shown in FIG.67A, FIG.68A, FIG.69A, and FIG.70A, the test strip 10B comprises the substrate 40A, the middle layer 90, the spacer layer 50A, and the cover layer 60A; FIG.67B illustrates a variation of the substrate 40A shown in FIG.67A; FIG.68B illustrates a variation of the substrate 40A shown in FIG.68A; FIG.69B illustrates a variation of the substrate 40A shown in FIG.69A; and FIG.70B illustrates a variation of the substrate 40A shown in FIG.70A. As shown in FIG.71A, FIG.72A, FIG.73A, and FIG.74A, the test strip 10B comprises the substrate 40A, the spacer layer 50A, and the cover layer 60A; FIG.71B illustrates test strip 10B of FIG.71A in a combined state; FIG.72B illustrates test strip 10B of FIG.72A in a combined state; FIG.73B illustrates test strip 10B of FIG.73A in a combined state; and FIG.74B illustrates test strip 10B of FIG.74A in a combined state.

In an embodiment of the present invention shown in FIG.63A to FIG.74B, in order to avoid the two reagents mixing with each other, a spacing bar 80 can be disposed between the first specimen path 12A and the second specimen path 12B (as shown in FIG.63A to FIG.63C, FIG.63H, FIG.64A to FIG.64C, FIG.64H, FIG.65B to FIG.65D, FIG.65H, FIG.66B to FIG.66D, FIG.66H, FIG.71A to FIG.74B); however, there can be other configurations for the present invention. In an embodiment of the present invention, the spacing bar 80 can be disposed on the first reference electrode 146A (as shown in FIG.63D, FIG.64D, FIG.65E, FIG.66E); besides, in order to keep the reaction reagent 16B away from affecting the flow time detection, the working electrode 147 can be extended into the first specimen path 12A (as shown in FIG.63E to FIG.63G, FIG.64E to FIG.64G, FIG.65A, FIG.65F, FIG.65G, FIG.66A, FIG.66F, FIG.66G, FIG.67A to FIG.68B, FIG.69A to FIG.70B). In an embodiment of the present invention, the working electrode 147 can be formed in a bar or a fork shape, and the spacing bar 80 is disposed on the working electrode 147 or between the fork of the working electrode 147.

In an embodiment of the present invention, when the first specimen path 12A of the test strip 10B is in series with the second specimen path 12B, the inlet end 122A of the first specimen path 12A can be disposed at a front end of the test strip 10B (as shown in FIG.63A to FIG.63H, FIG.64A to FIG.64H, FIG.69A to FIG.70B, FIG.71A to FIG.72B) or a side of the test strip 10B (as shown in FIG.65A to FIG.65H, FIG.66A to FIG.66H, FIG.67A to FIG.68B, FIG.73A to FIG.74B).

In an embodiment of the present invention, when the first specimen path 12A is in series with the second specimen path 12B, the test strip 10B can also comprises a through hole 70B communicating with the discharge end 124B of the second specimen path 12B (as shown in FIG.63A, FIG.64A, FIG.69A, FIG.70A, FIG.71A to FIG.72B); and the first specimen path 12A can have the same width as that of the second specimen path 12B (as shown in FIG.63A, FIG.65A, FIG.67A, FIG.69A, and FIG.71A to FIG.74B); or, the first specimen path 12A has a width smaller than that of the second specimen path 12B(as shown FIG.64A, FIG.66A, FIG.68A. and FIG.70A).

In an embodiment of the present invention, the first electrode set 14A of the test strip 10B further comprises a second electrode 144A. When the specimen 30A flows through the second electrode 144A and the first reference electrode 146A, a third impulse signal is generated, wherein the third impulse signal is used with the first impulse signal and the second impulse signal to obtain the flow time of the specimen 30A, thereby obtaining the concentration of the analyte. As shown in FIG.63H, FIG.64H, FIG.65H, and FIG.66H, the second electrode 144A is disposed between the first electrode 142A and the working electrode 147; however, the present invention can have other configurations. By using the second electrode 144A, at least two sets of flow time values are obtained; if the two sets of flow time values are very different from each other, then an error alert is issued to a user.

As shown in FIG.63A to FIG.74B, in an embodiment of the present invention, when the first specimen path 12A is in series with the second specimen path 12B, the first electrode set 14A and the second electrode set 14B of the test strip 10B can be disposed as the following configurations, however, the present invention can have other configurations as well.
1 The first electrode set 14A comprises the first electrode 142A and the first reference electrode 146A, and the second electrode set 14B comprises the working electrode 147 and the second reference electrode 146B.
2, The first electrode set 14A comprises the first electrode 142A, the second electrode 144A, and the first reference electrode 146A, and the second electrode set 14B comprises the working electrode 147 and the second reference electrode 146B. The first reference electrode 146A and the second reference electrode 146B can be the same electrode or different electrodes.
3, The first electrode set 14A comprises the first electrode 142A, the second electrode 144A, and the first reference electrode 146A, and the second electrode set 14B comprises the working electrode 147, the detector electrode 149, and the second reference electrode 146B.

Additionally, in an embodiment of the present invention, the first reference electrode 146A and the second reference electrode 146B of the test strip 10B can be disposed on the lower surface of the cover layer 60A (shown in FIG.71A to FIG.74B) to form a stack configuration, wherein the first reference electrode 146A and the second reference electrode 146B can be the same electrode (as showin in FIG.71A, FIG.71B, FIG.73A, and FIG.73B).

As shown in FIG.67A and FIG.70B, in an embodiment of the present invention, the test strip 10B can also comprise a middle layer 90 disposed between the substrate 40A and the spacer layer 50A to separate the first specimen path 12A and the second specimen path 12B. The middle layer 90 can be formed by screen printing an insulating layer or by attaching a spacer layer.

As shown in FIG.63A to FIG.74B, in an embodiment of the present invention, when the first specimen path 12A is in series with the second specimen path 12B, the first electrode set 14A of the test strip 10B can be disposed in various configurations; the second electrode set 14B can be disposed in various configurations as well; the arrangement of the first electrode set 14A relative to the second electrode set 14B can be varied; the redox reagent 16A can be disposed in various configuration; the reaction reagent 16B can be disposed in various configurations; the inlet end 122A of the first specimen path 12A and the inlet end 122B of the second specimen path 12B can be disposed in various configurations; and the first specimen path 12A and the second specimen path 12B can be arranged in other configurations. Additionally, in an embodiment of the present invention, the first electrode set 14A further comprises a second electrode 144A, when the specimen 30A flows through the second electrode 144A and the first reference electrode 146A, a third impulse signal is generated, wherein the third impulse signal is used with the first impulse signal and the second impulse signal to obtain the flow time of the specimen.

Finally, the present invention provides a detection method working with an electrochemical instrument to detect a specimen, thereby obtaining a flow time of the specimen and using the flow time to correct the concentration of the analyte of the specimen. In the following, the detecting device 1, the test strip 10, 10A and 10B are used to understand the detection method of the present invention; however, the detection method of the present invention can also use devices other than the detecting device 1, the test strip 10, 10A and 10B.

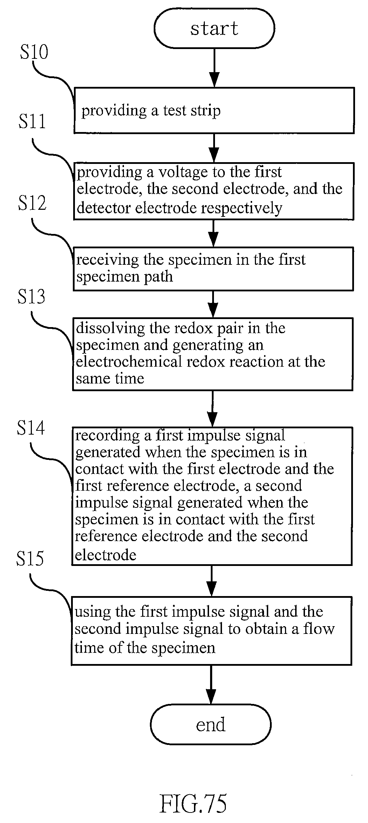

As shown in FIG.75, in an embodiment of the present invention, the present invention provides a detection method. First, the present invention proceeds to step S10: providing a test strip. In an embodiment of the present invention, the test strip comprises: a first specimen path, a first electrode set, a redox reagent, a second specimen path, a second electrode set, and a reaction reagent. The first electrode set comprises a first electrode, a second electrode, and a first reference electrode; a second electrode set comprises a working electrode, a detector electrode, and a second reference electrode. Since the structure of the test strip has been illustrated in detail with the example of test strip 10A, it will not be further described for the sake of brevity.

Then the method proceeds to step S 11: providing a voltage to the first electrode, the second electrode, and the third electrode respectively; step S12: receiving the specimen in the first specimen path; step S 13: dissolving the redox pair in the specimen and generating an electrochemical redox reaction at the same time; step S 14: recording a first impulse signal generated when the specimen is in contact with the first electrode and the first reference electrode, a second impulse signal generated when the specimen is in contact with the first reference electrode and the second electrode; and step S 15: using the first impulse signal and the second impulse signal to obtain a flow time of the specimen.

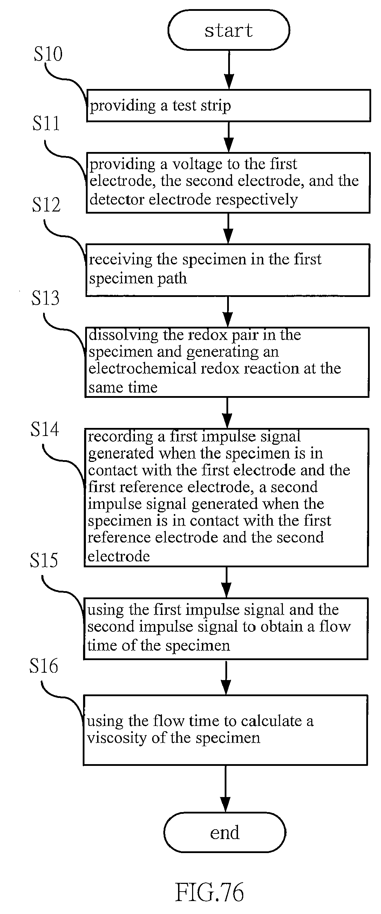

As shown in FIG.76, after step S 15, the detection method of the present invention can proceed to step S16: using the flow time to calculate a viscosity of the specimen.

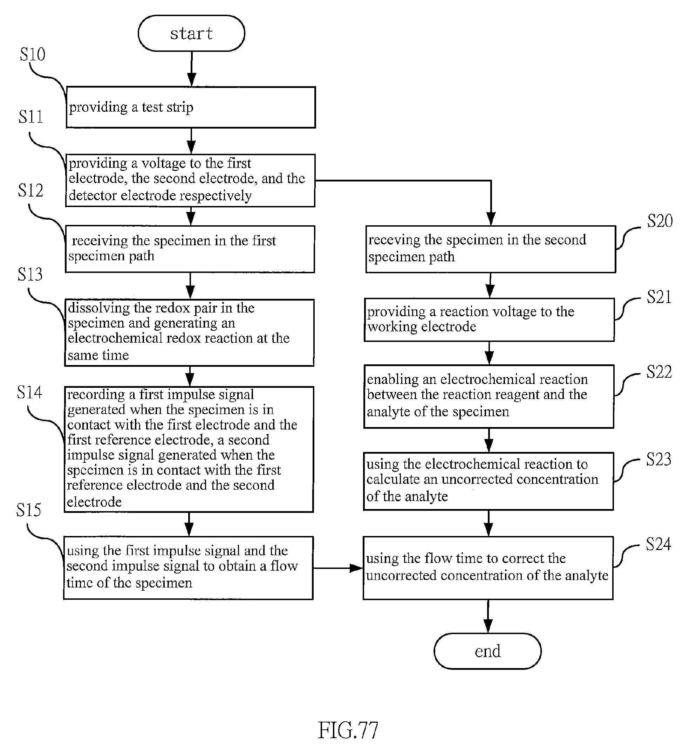

As shown in FIG.77, apart from the steps S10 to S 15, the method can proceed to step S20 to S24 after the step S11 of providing a voltage to the first electrode, the second electrode, and the third electrode respectively is performed; thereby obtaining a corrected concentration of the analyte. As shown in FIG.77, the detection method of the present invention also proceeds to step S20: receving the specimen in the second specimen path; step S21: providing a reaction voltage to the working electrode; step S22: enabling an electrochemical reaction between the reaction reagent and the analyte of the specimen; step S23: using the electrochemical reaction to calculate an uncorrected concentration of the analyte; and step S24: using the flow time to correct the uncorrected concentration of the analyte.

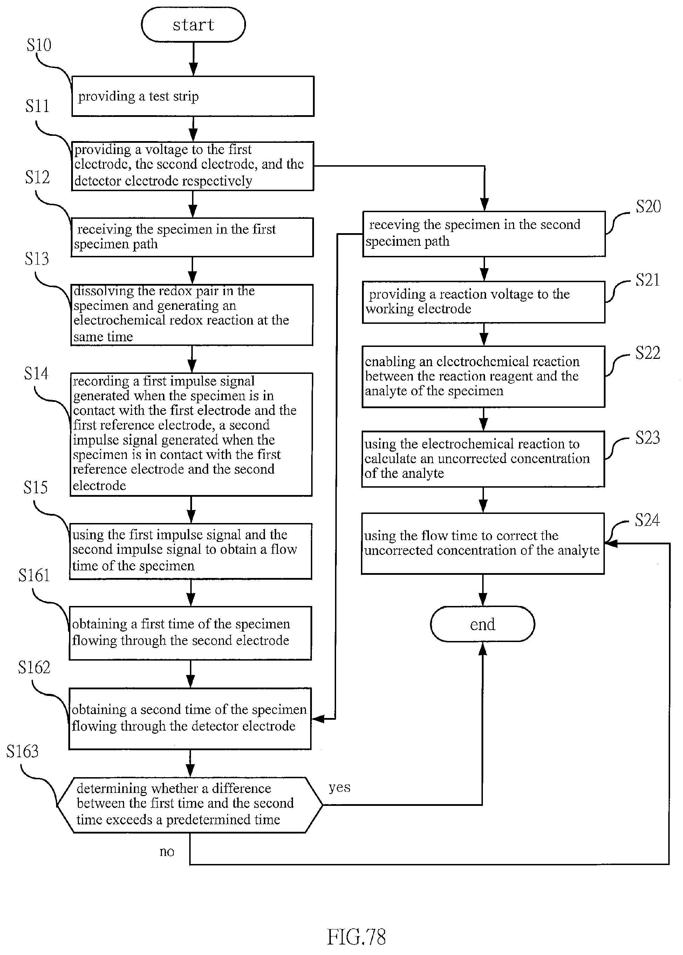

In an embodiment of the present invention, the specimen enters the first specimen path and the second specimen path at the same time; therefore, the present invention can compare the time of the specimen flowing through the first specimen path with the time of the specimen flowing through the second specimen path to make sure whether the detecting device is operating normally. Hence, as shown in FIG.78, after step S 15, the detection method of the present invention proceeds to step S161: obtaining a first time of the specimen flowing through the second electrode; after step S20, the method proceeds to step S162: obtaining a second time of the specimen flowing through the detector electrode; then the method proceeds to step S163: determining whether a difference between the first time and the second time exceeds a predetermined time. If the difference between the first time and the second time exceeds a predetermined time, then the specimen does not flow normally, the detection is invalid, and the detection method is terminated; if the difference between the first time and the second time does not exceed a predetermined time, then the specimen flows normally, the detection is valid, and the method proceeds to step S24: using the flow time to correct the uncorrected concentration of the analyte.

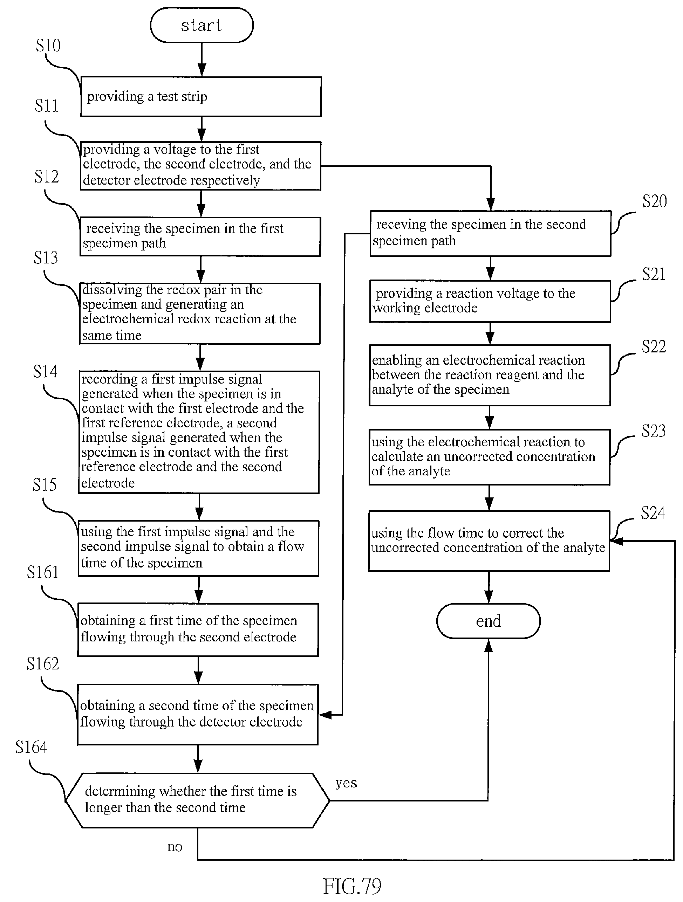

In an embodiment of the present invention, the specimen enters the first specimen path and the second specimen path at the same time; therefore, the present invention can compare the time of the specimen flowing through the first specimen path with the time of the specimen flowing through the second specimen path to make sure whether the detecting device is operating normally. Hence, as shown in FIG.79, after step S 15, the detection method of the present invention proceeds to step S161: obtaining a first time of the specimen flowing through the second electrode; after step S20, the method proceeds to step S162: obtaining a second time of the specimen flowing through the detector electrode; then the method proceeds to step S164: determining whether the first time is longer than the second time. If the first time should be equal to or shorter than the second time under normal operation, then the specimen does not flow normally, the detection is invalid, and the detection method is terminated; if the first time is shorter than the second time, then the specimen flows normally, the detection is valid, and the method proceeds to step S24: using the flow time to correct the uncorrected concentration of the analyte.

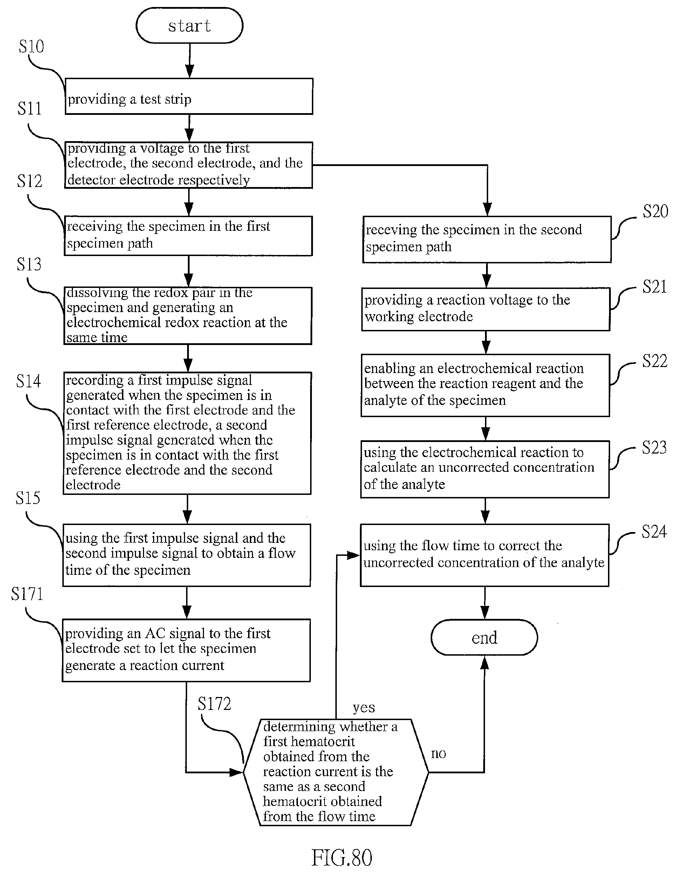

Furthermore, as shown in FIG.80, in order to obtain a more accurate concentration of the analyte, after step S 15, the detection method of the the present invention proceeds to step S 171: providing an AC signal to the first electrode set to let the specimen generate a reaction current; step S 172 determining whether a first hematocrit obtained from the reaction current is the same as a second hematocrit obtained from the flow time. If the first hematocrit is very different from the second hematocrit, then the specimen does not flow normally, the detection is invalid, and the detection method is terminated; if the first hematocrit is close to the second hematocrit, then the specimen flows normally, the detection is valid, and the method proceeds to step S24: using the flow time to correct the uncorrected concentration of the analyte. Since using the AC signal to compensate the concentration of the analyte is well known in the art, it will not be further described for the sake of brevity.

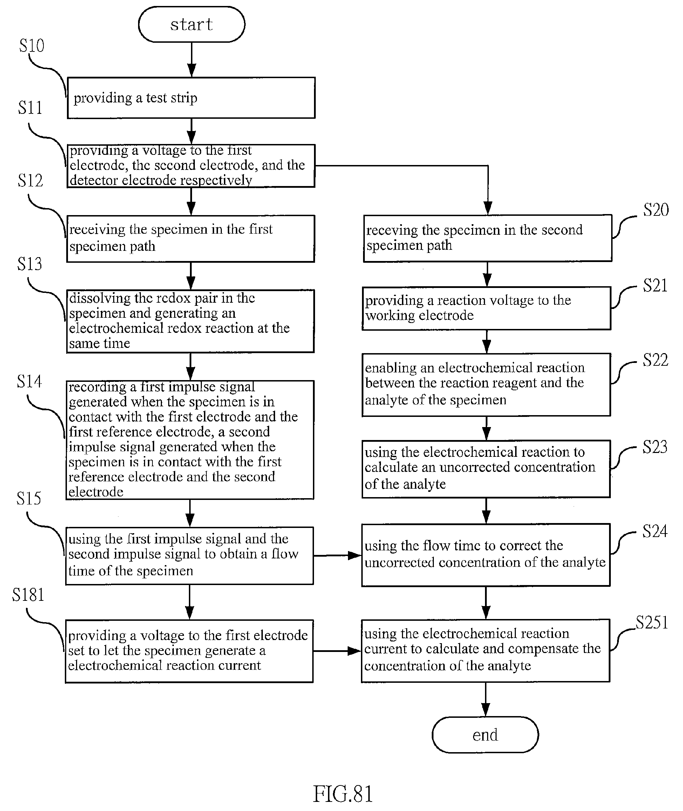

Furthermore, as shown in FIG.81, in order to obtain a more accurate concentration of the analyte, after step S 15, the detection method of the the present invention proceeds to step S181: providing a voltage to the first electrode set to let the specimen generate an electrochemical reaction current. Thereafter, in addition to step S24: using the flow time to correct the uncorrected concentration of the analyte, the method further proceeds to step S251: using the electrochemical reaction current to calculate and compensate the concentration of the analyte. Since the step of using the electrochemical reaction current to calculate and compensate the concentration of the analyte is well known in the art, t will not be further described for the sake of brevity.

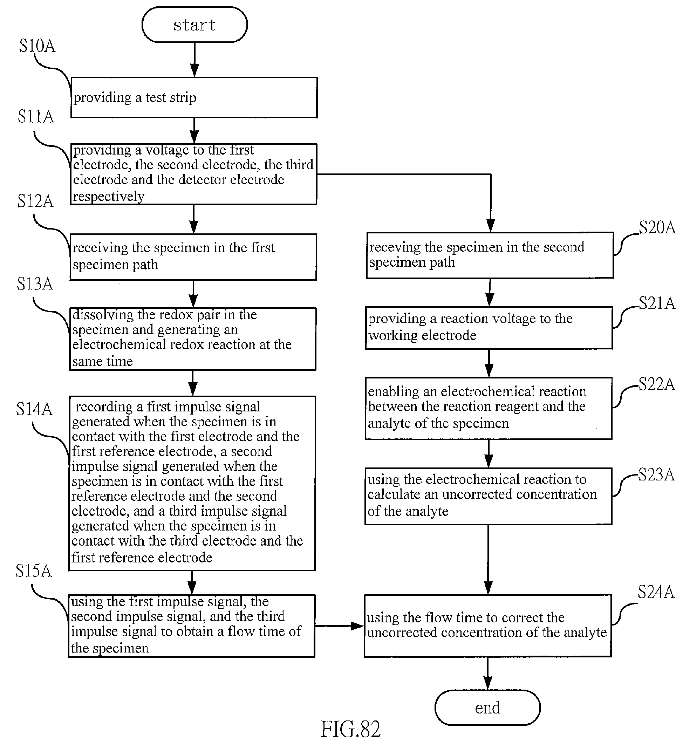

In an embodiment of the present invention, the present invention provides a test strip having a plurality of test strip for accurately detecting the flow time. As showin in FIG. 82, in an embodiment of the present invention, the present invention further provides a detection method, which first proceeds to step S10A: providing a test strip. In an embodiment of the present invention, the test strip comprises: a first specimen path, a first electrode set, a redox reagent, a second specimen path, a second electrode set and a reaction reagent. The first electrode set comprises a first electrode, a second electrode, a third electrode, and a first reference electrode; the second electrode set comprises a working electrode, a detector electrode, and a second reference. Since the structure of the test strip having the third electrode is described with the example of the test strip 10A, it will not be further described for the sake of brevity.

Then the method proceeds to step S11A: providing a voltage to the first electrode, the second electrode, the third electrode, and the detector electrode respectively; step S12A: receiving the specimen in the first specimen path; step S 13A: dissolving the redox pair in the specimen and generating an electrochemical redox reaction at the same time; step S 14A: recording a first impulse signal generated when the specimen is in contact with the first electrode and the first reference electrode, a second impulse signal generated when the specimen is in contact with the first reference electrode and the second electrode, and a third impulse signal generated when the specimen is in contact with the third electrode and the first reference electrode; step S15A: using the first impulse signal, the second impulse signal, and the third impulse signal to obtain a flow time of the specimen.

As shown in FIG.82, in addition to step S10A to S15A and after step S11A, the detection method proceeds to step S20A to S24A for obtaining the corrected concentration of the analyte. As shown in FIG.82, the detection method of the present invention proceeds to step S20A: receiving the specimen in the second specimen path; step S21A: providing a reaction voltage to the working electrode; step S22A: enabling an electrochemical reaction between the reaction reagent and the analyte of the specimen; step S23A: using the electrochemical reaction to calculate an uncorrected concentration of the analyte; and step S24A: using the flow time to correct the uncorrected concentration of the analyte.

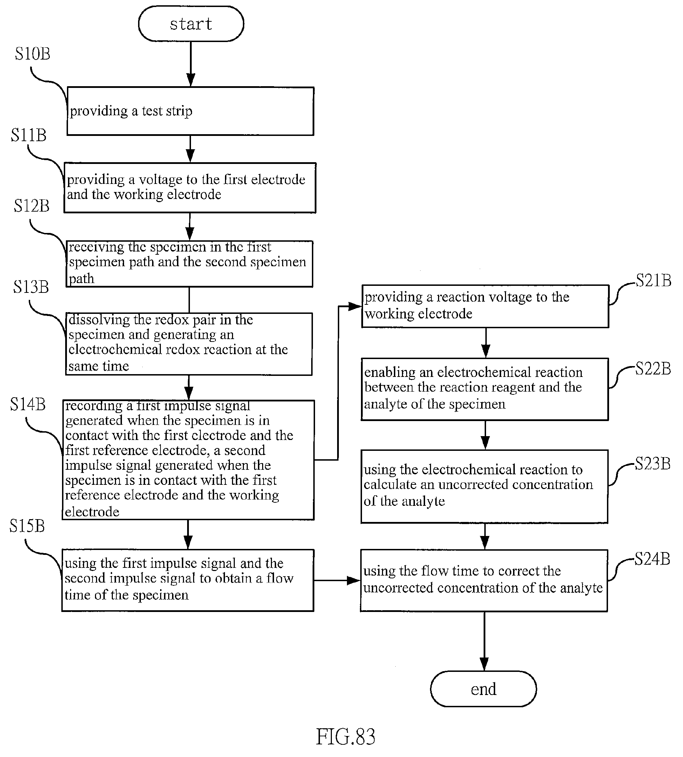

In an embodiment of the present invention, the present invention can be applied to the test strip having series specimen paths. As shown in FIG.83, in an embodiment of the present invention, the detection method of the present invention first proceeds to step S10B: providing a test strip. In an embodiment of the present invention, the test strip comprises: a first specimen path, a first electrode set, a redox reagent, a second specimen path, a second electrode set, and a reaction reagent. The first electrode set comprises a first electrode, a second electrode, and a first reference electrode; a second electrode set comprises a working electrode and a second reference electrode; the first specimen path is in series with the second specimen path. Since the structure of the test strip in a series mode has been illustrated in detail with the example of test strip 10B, it will not be further described for the sake of brevity.

Then the method proceeds to step S11B: providing a voltage to the first electrode and the working electrode; step S12B: receiving the specimen in the first specimen path and the second specimen path, wherein the specimen first passes through the first specimen path and then the second specimen path; step S 13B: dissolving the redox pair in the specimen and generating an electrochemical redox reaction at the same time; step S 14B : recording a first impulse signal generated when the specimen is in contact with the first electrode and the first reference electrode, a second impulse signal generated when the specimen is in contact with the first reference electrode and the working electrode; and step S15B: using the first impulse signal and the second impulse signal to obtain a flow time of the specimen.

As shown in FIG.83, in addition to step S10B to S15B, after step S14B, the detection method of the present invention proceeds to step S21B to S24B to obtain a corrected concentration of the analyte. As shown in FIG.83, the detection method of the present invention proceeds to step S21B: providing a reaction voltage to the working electrode; step S22B: enabling an electrochemical reaction between the reaction reagent and the analyte of the specimen; step S23B: using the electrochemical reaction to calculate an uncorrected concentration of the analyte; and step S24B: using the flow time to correct the uncorrected concentration of the analyte. In the embodiment of the present invention, the working electrode is operated as a time detecting electrode at the same time. At the beginning, the electrochemical instrument provides a voltage to the working electrode for detecting the second impulse signal; when the second impulse signal is received, the electrochemical instrument will shut down the voltage immediately to provide a reaction voltage to the working electrode for an electrochemical reaction.

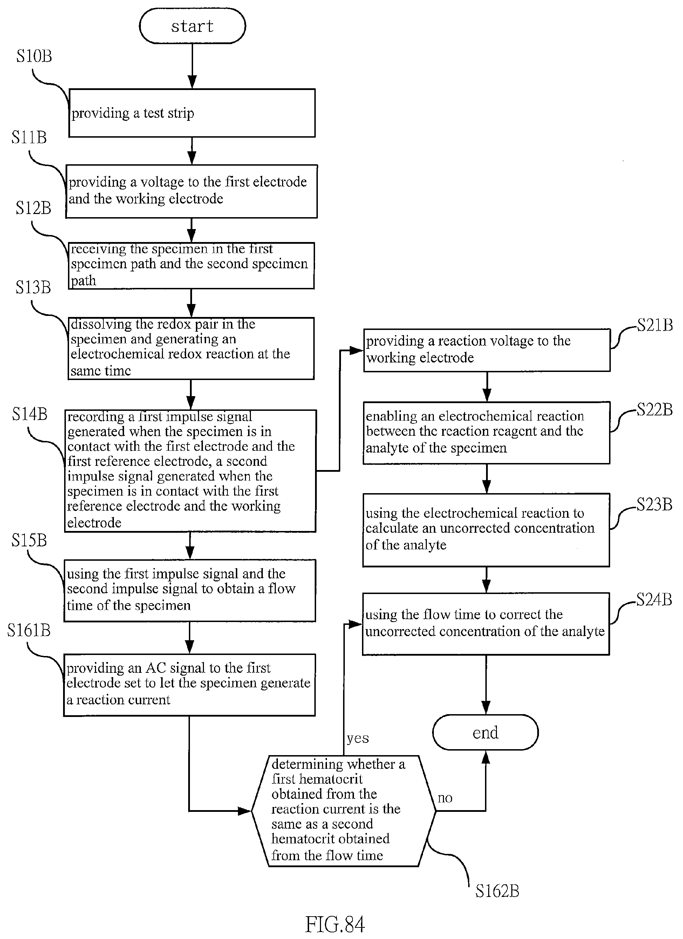

Furthermore, as shown in FIG. 84, in order to obtain a more accurate concentration of the analyte, after step S 15B, the detection method of the the present invention proceeds to step S161B: providing an AC signal to the first electrode set to let the specimen generate a reaction current; step S162B determining whether a first hematocrit obtained from the reaction current is the same as a second hematocrit obtained from the flow time. If the first hematocrit is very different from the second hematocrit, then the specimen does not flow normally, the detection is invalid, and the detection method is terminated; if the first hematocrit is close to the second hematocrit, then the specimen flows normally, the detection is valid, and the method proceeds to step S24B: using the flow time to obtain the corrected concentration of the analyte. Since using the AC signal to compensate the concentration of the analyte is well known in the art, it will not be further described for the sake of brevity.

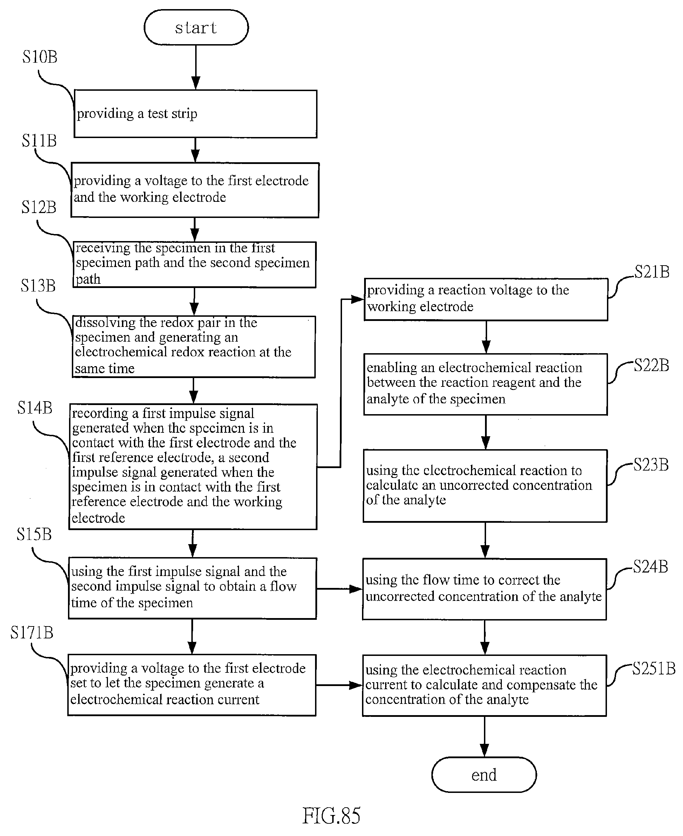

Furthermore, as shown in FIG.85, in order to obtain a more accurate concentration of the analyte, after step S15B, the detection method of the the present invention proceeds to step S171B: providing a voltage to the first electrode set to let the specimen generate a electrochemical reaction current. Thereafter, in addition to step S24B: using the flow time to obtain the corrected concentration of the analyte, the method further proceeds to step S251B: using the electrochemical reaction current to calculate and compensate the concentration of the analyte. Since the step of using the electrochemical reaction current to calculate and compensate the concentration of the analyte is well known in the art, it will not be further described for the sake of brevity.

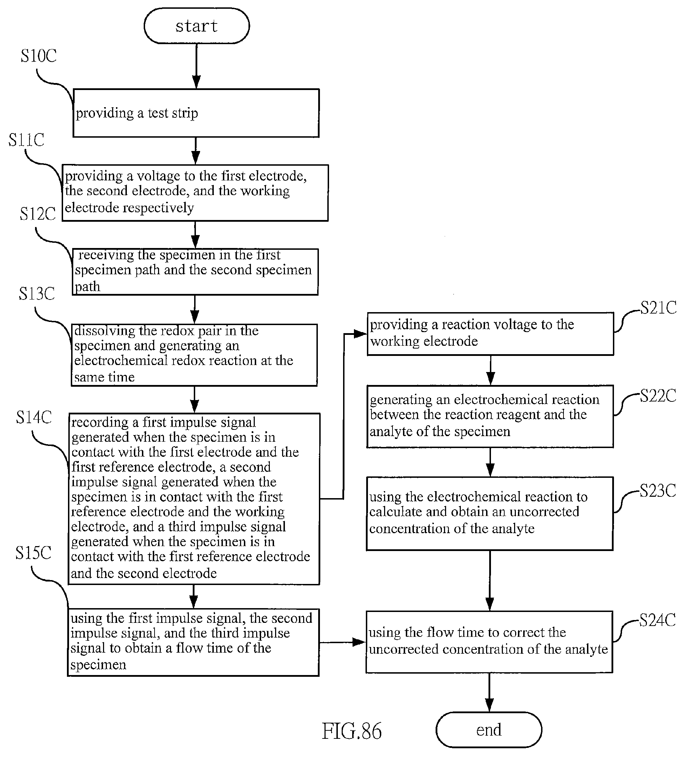

In an embodiment of the present invention, the present invention provides a test strip having series specimen paths and a plurality of electrodes for accurately detecting the flow time. As showin in FIG.86, in an embodiment of the present invention, the present invention further provides a detection method, which first proceeds to step S10C: providing a test strip. In an embodiment of the present invention, the test strip comprises: a first specimen path, a first electrode set, a redox reagent, a second specimen path, a second electrode set and a reaction reagent. The first electrode set comprises a first electrode, a second electrode, and a first reference electrode; the second electrode set comprises a working electrode and a second reference electrode; and the first specimen path is in series with the second specimen path. Since the structure of the test strip having series specimen paths and the second electrode is described with the example of the test strip 10B, it will not be further described for the sake of brevity.

Then the method proceeds to step S 11 C: providing a voltage to the first electrode, the second electrode, and the working electrode respectively; step S12C: receiving the specimen in the first specimen path and the second specimen path, wherein the specimen first passes through the first specimen path and then the second specimen path; step S13C: dissolving the redox pair in the specimen and generating an electrochemical redox reaction at the same time; step S 14C: recording a first impulse signal generated when the specimen is in contact with the first electrode and the first reference electrode, a second impulse signal generated when the specimen is in contact with the first reference electrode and the working electrode, and a third impulse signal generated when the specimen is in contact with the first reference electrode and the second electrode; and step S15C: using the first impulse signal, the second impulse signal, and the third impulse signal to obtain a flow time of the specimen.

As shown in FIG.86, in addition to steps S10C to S15C, after step 14C, the detection method of the present invention can also proceed to step S21 C to step S24C to obtain a corrected concentration of the analyte. As shown in FIG.86, the detection method of the present invention proceeds to step S21C: providing a reaction voltage to the working electrode; step S22C: generating an electrochemical reaction between the reaction reagent and the analyte of the specimen; step S23C: using the electrochemical reaction to calculate and obtain an uncorrected concentration of the analyte; and step S24C: using the flow time to correct the uncorrected concentration of the analyte.

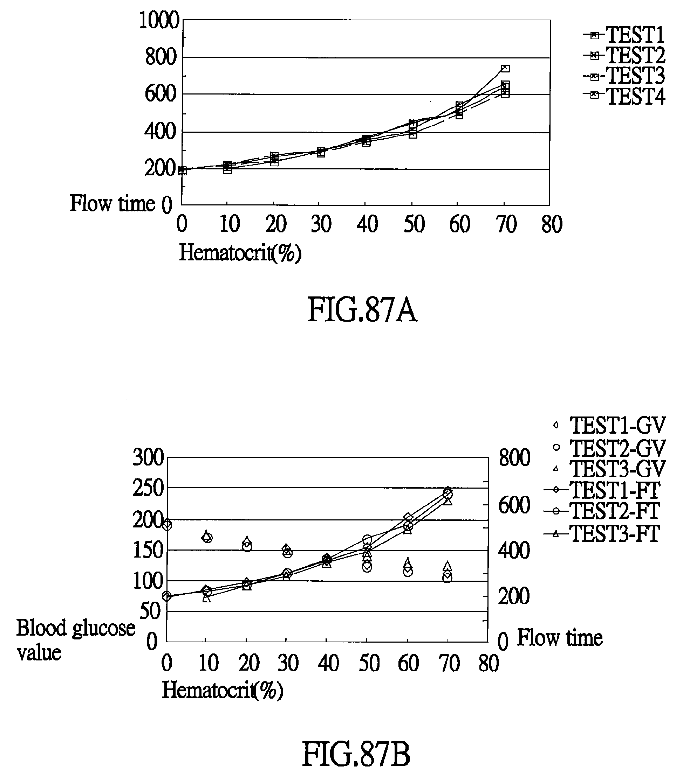

As above, when the detecting device 1 is used as a detecting device for detecting blood glucose, the detecting device can accurately obtain a flow time and a viscosity of the blood of the specimen, thereby obtaining a value of the hematocrit. FIG.87A to FIG.87B shows the results of using venous bloods of different hematocrits as different viscosity conditions versus blood glucose values. From the experimental result, the experiment is reproducible with the coeffecient variation (CV) less than 10. When the viscosity increases, the flow time is longer; meanwhile, when the viscosity goes higher, the blood glucose value drops. As can be seen from the result, the variation of blood glucose values due to different viscosities can be corrected by the flow time obtained in the present invention, thereby removing the interference factors caused by viscosity and obtaining accurate blood glucose values.

It is noted that the above-mentioned embodiments are only for illustration. It is intended that the present invention cover modifications and variations of this invention provided they fall within the scope of the following claims and their equivalents. Therefore, it will be apparent to those skilled in the art that various modifications and variations can be made to the structure of the present invention without departing from the scope or spirit of the invention.

## Claims

1. A test strip (10) adapted to be electrically connected with an electrochemical instrument (20) for detecting a specimen (30), whereby the electrochemical instrument (20) is used for obtaining a flow time of the specimen (30), **characterized, in that** the test strip (10) comprising:
a specimen path (12) comprising an inlet end (122) and a discharge end (124);
an electrode set (14) having at least a portion thereof disposed in the specimen path (12), the electrode set (14) at least comprising a first electrode (142), a second electrode (144) and a reference electrode (146);
a redox reagent (16) disposed in the specimen path (12), the redox reagent (16) at least comprising a redox pair, wherein the redox pair comprises potassium ferricyanide and potassium ferrocyanide;
when the specimen (30) enters the specimen path (12), the redox pair dissolves and generates an electrochemical redox reaction for generating a first impulse signal when the specimen (30) is in contact with the first electrode (142) and the reference electrode (146) and generating a second impulse signal when the specimen (30) is in contact with the second electrode (144) and the reference electrode (146), wherein the first electrode (142), the second electrode (144), and the reference electrode (146) are positioned such that the entering specimen (30) electrically connects the first electrode (142) with the reference electrode (146) for generating the first impulse signal, and the further-flowing specimen (30) later electrically connects the second electrode (144) with the reference electrode (146) for generating the second impulse signal, thereby the electrochemical instrument (20) can calculate the flow time of the specimen (30) according to the first impulse signal and the second impulse signal.

2. The test strip (10) as claimed in Claim 1, wherein the electrode set (14) further comprises a third electrode (148), when the specimen (30) flows through the third electrode (148) and the reference electrode (146), a third impulse signal is generated and is used with the first impulse signal and the second impulse signal to obtain the flow time of the specimen (30).

3. The test strip (10) as claimed in Claim 2, wherein the third electrode (148) is disposed near the first electrode (142).

4. The test strip (10) as claimed in Claim 2, wherein the third electrode (148) is disposed between the first electrode (142) and the second electrode (144).

5. The test strip (10) as claimed in Claim 1, wherein the first electrode (142) is disposed near the inlet end (122) of the specimen path (12), the second electrode (144) is disposed near the discharge end (124) of the specimen path (12).

6. The test strip (10) as claimed in Claim 1, wherein the redox reagent covers at least a portion of the electrode set (14).

7. The test strip (10) as claimed in Claim 1, wherein the redox reagent is disposed near the inlet end (122) of the specimen path (12).

8. The test strip (10) as claimed in Claim 1 further comprising:
a substrate (40) for disposing the electrode set (14) thereon;
a spacer layer (50) covering the substrate (40) and exposing a portion of the electrode set (14); and
a cap layer (60) covering the spacer layer (50) to form the specimen path (12).

9. The test strip (10) as claimed in Claim 1 further comprising:
a substrate (40) for disposing the first electrode (142) and the second electrode (144) thereon;
a spacer layer (50) covering the substrate (40) and exposing a portion of the first electrode (142) and the second electrode (144); and
a cap layer (60) covering the spacer layer (50) to form the specimen path (12), the cap layer (60) having the reference electrode (146) disposed at a lower surface of the cap layer (60).

10. The test strip (10) as claimed in Claim 8 further comprising a through hole (70), wherein the through hole (70) penetrates through the substrate (40), the spacer layer (50), and the cap layer (60) to communicate with the discharge end (124) of the specimen path (12).

11. The test strip (10) as claimed in Claim 9 further comprising a through hole (70), wherein the through hole (70) penetrates through the substrate (40), the spacer layer (50), and the cap layer (60) to communicate with the discharge end (124) of the specimen path (12).

12. The test strip (10) as claimed in Claim 1 further comprising a through hole (70) communicating with the discharge end (124) of the specimen path (12).

13. The test strip (10) as claimed in Claim 1, wherein the inlet end (122) of the specimen path (12) is disposed at a front end or a side of the test strip (10).

14. A detection method using an electrochemical instrument (20) to detect a specimen (30), the detection method comprising the following steps:
providing the test strip (10) as claimed in any of the Claims 1- 13;
providing a voltage to the electrode set (14);
receiving the specimen (30) in the specimen path (12);
dissolving the redox pair in the specimen (30) and generating an electrochemical redox reaction at the same time;
recording a first impulse signal generated when the specimen (30) is in contact with the first electrode (142) and the reference electrode (146), and a second impulse signal generated when the specimen (30) is in contact with the second electrode (144) and the reference electrode (146); and
using the first impulse signal and the second impulse signal to obtain a flow time of the specimen (30) according to the first impulse signal and the second impulse signal.

15. The detection method as claimed in Claim 14, further comprises:
providing the test strip (10) as claimed in any of the Claims 2-4;
recording the third impulse signal generated when the specimen (30) is in contact with the third electrode (148) and the reference electrode (146); and
using the first impulse signal, the second impulse signal, and the third impulse signal to obtain the flow time of the specimen (30) according to the first impulse signal, the second impulse signal, and the third impulse signal.
